# EUROPEAN PATENT APPLICATION

(11) **EP 4 699 647 A1**
(43) Date of publication of application: **25.02.2026**
(21) Application number: 24195906.3
(22) Date of filing: 22.08.2024
(51) Int. Cl.: A61N 2/02, A61N 2/00

(54) **CIRCUITS, APPARATUSES, AND METHODS FOR MAGNETIC STIMULATION**

(71) Applicant: Fotona d.o.o., 1000 Ljubljana (SI)
(72) Inventor: LUKAC, Matjaz, 1000 Ljubljana (SI); KATARINCIC, Peter, 1290 Grospulje (SI); HRELJAC, Irena, Lubljana (SI); GASPAR, Adrian, Mendoza 5503 (AR); VASILEVA, Teodora, 38444 Wolfsburg (DE)
(74) Representative: Bardehle Pagenberg Partnerschaft mbB Patentanwälte Rechtsanwälte

(57) **Abstract**

The present invention relates to an apparatus for magnetic stimulation. The apparatus comprises a first element adapted to receive at least a part of a body of a patient and a first set of one or more magnetic coils arranged at the first element. In particular, the first element is adjustable such as to move relative to the first set of one or more magnetic coils.

## Description

### Field of the invention

Described herein are a circuit for generating a time-varying magnetic field for magnetic stimulation and applications of such a circuit as well as other circuits, e.g., in an apparatus and/or method for reducing inflammation and/or in a method for treating erectile dysfunction and/or premature ejaculation.

### Technical background

Magnetic stimulation refers to a non-invasive method of delivering a time-varying magnetic field to the periphery of a human or an animal body. Magnetic stimulation can be considered a (still) novel, painless and easy approach for treating many neurological and musculoskeletal conditions.

The physical principle of magnetic stimulation is based on a time-varying current flow passing through an electromagnetic coil, resulting in an equally time-varying (e.g., pulsed) magnetic field around the coil. When a pulse of the magnetic field passes into the body, the pulse will induce a voltage difference between spatially separated points in and/or on the body. This voltage difference yields an electric field and thus induces electrons to flow between the spatially separated points. Different from electrical stimulation, magnetic stimulation does not need a traverse of electrical current through electrodes, skin and tissue. The magnetic field acts as the vehicle or medium to induce ions to flow, and it does not stimulate the nervous tissue itself. However, once the ion flow is induced, the mechanisms of electrical and magnetic stimulation are the same at the neural level, both in particular rely on axon depolarization and the initiation of an action potential.

Such stimulation by means of a time-varying magnetic field provides for many advantages. Firstly, a magnetic field can pass any medium, even a vacuum, without any attenuation of energy. This allows for penetration into deep tissue such as spinal nerve roots or deep muscles. Consequently, no mechanical contact is necessary, making magnetic stimulation applicable to patients with extreme hypersensitivity or allodynia to skin contact. Further, as a magnetic field can pass through clothing, the patient does not need to undress. Moreover, due to no charged particles being injected into the skin and superficial tissue, and the weak recruitment ability of cutaneous sensory afferent fiber, magnetic stimulation rarely causes pain in clinical practice.

Magnetic stimulation therapies may be divided into two basic categories. In nerve stimulation therapies, such as transcranial magnetic stimulation or spinal cord stimulation therapies, the desired end effect is nerve stimulation. Meanwhile, in muscle contraction stimulation therapies, the desired end effect is involuntary muscle contraction caused by the electric current induced in motor neurons. Both categories of magnetic stimulation therapies rely on inducing electric current in the body, resulting in the stimulation of nerves through axon depolarization and the initiation of an action potential. It should be noted that in many therapies a combination of both nerve stimulation therapeutical effects and muscle contraction stimulation therapeutical effects may be present.

Magnetic stimulation therapies have a variety of applications. The indications of magnetic nerve stimulation therapies, in particular spinal cord stimulation therapies, include relief of pain associated with failed back surgery syndrome, refractory angina pectoris, peripheral vascular disease, and complex regional pain syndrome. As to transcranial magnetic stimulation therapies, indications include depression, obsessive-compulsive disorder, migraines, anxiety and other brain-related conditions. Concerning muscle contraction stimulation therapies, the indications include muscle toning, firming and strengthening, improving muscle performance, fat reduction, urinary incontinence, stimulating neuromuscular tissue for bulk muscle excitation in the legs or arms for rehabilitative purposes, e.g., relaxation of muscle spasm, prevention or retardation of disuse atrophy, increasing local blood circulation, muscle re-education, immediate post-surgical stimulation of calf muscles to prevent venous thrombosis as well as maintaining or increasing a range of motion. It should be clear from the foregoing that, even though the expressions "therapy" and "indication" have been used (and will occasionally be used throughout the following), magnetic stimulation does not only serve therapeutic (in the narrower sense), but also, additionally or alternatively, cosmetic ends.

Clearly, different indications may require different types/embodiments of magnetic stimulation. As magnetic stimulation is based on the application of a time-varying (e.g., pulsed) magnetic field, a given treatment may be characterized by parameters associated with the corresponding magnetic field. For example, a strength of a therapy may be characterized by three physical critical parameters: first, the (maximum) amplitude of pulses of the magnetic field delivered, which is typically defined in Tesla (T); secondly, the pulse rate at which pulses of the magnetic field are delivered, which is typically defined in Hertz (Hz); thirdly, the surface area of the volume irradiated, which is typically defined in cm².

From a physical and/or engineering perspective, a critical and fundamental problem in magnetic stimulation is the creation of a time-varying (also termed alternating) magnetic field. A magnetic field B is formed as a natural physical phenomenon when a resonant electric current flows through the electromagnetic coil of an applicator. In its most primitive form, this can be achieved by a LC circuit (also termed resonant circuit) composed of an electromagnetic coil of inductance L and a capacitor of capacitance C. The inductance L and capacitance C determine the resonant frequency fᵣ of the circuit. An ideal resonant circuit, i.e., a resonant circuit without ohmic losses, would resonate (e.g., oscillate) indefinitely. However, in reality, due to losses, a resonant circuit requires a continuous supply of power as well as a continuous (re-)triggering by means of a switch to initiate oscillations.

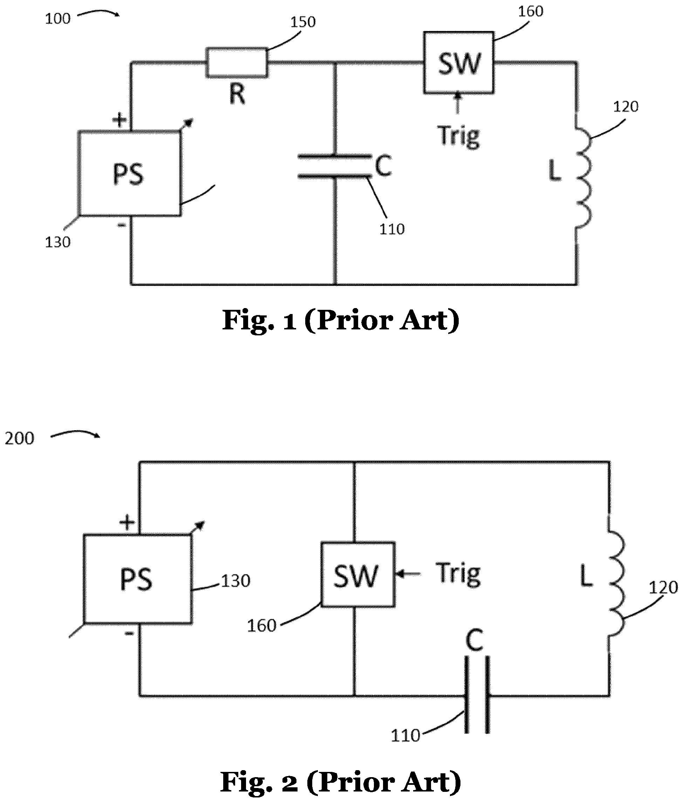

Referring to Fig. 1, in order for an LC circuit 100 to be functional, the LC circuit 100 must be equipped with a power supply PS 130 and a (triggering) switch SW 160. A circuit comprised of such elements forms the basic circuit for generating resonant LC oscillations where the power supply PS 130 delivers the initial electrical charge onto the capacitor C 110. Once the switch SW 160 is triggered, the capacitor C 110 gets dis- and recharged in a series of sine-shaped currents of frequency fᵣ (also called resonant frequency, whose inverse corresponds to an oscillation period, or resonant period, tᵣ) wherein the number of sine-shaped currents/(resonant) oscillations depends on the number of triggering pulses delivered and how quickly the capacitor charge gets depleted. This is because typically the (resonant) oscillations are much faster than the power supply PS 130's rate of re-charging of the capacitor C 110. A series of M sine-shaped currents in total results in an emission of M magnetic field oscillations, altogether constituting a magnetic field pulse. Provided there is at least a minimum charging time t_{c} needed by the power supply PS 130 to re-charge the capacitor C 110 in between pulses, magnetic field pulses can be delivered continuously up to a maximum repetition rate of 1/t_{c}.

In the state of the art, circuits for generating a time-varying magnetic field commonly include a resistor, cf. resistor R 150 in Fig. 1. The resistor R 150 serves as a current-reduction element limiting the initial charging current onto the capacitor C 110, and as an over-current protection for when a reverse(d) polarity (e.g., a current reverse in polarity to the initial charging current) occurs. This reverse(d) polarity hails from the electromagnetic coil L 120 opposing a change in the electric current flowing through it. Therefore, during a second half of the LC resonance, the polarity of (e.g., the voltage across) the capacitor is reverse(d) as compared to the power supply PS 130. This (voltage of) reverse(d) polarity affects and may damage the diodes which are located in the power supply PS 130's output stage. Consequently, the role of the resistor R 150 is to serve as an over-current protection for the power supply PS 130's diodes in that it limits the reverse(d) polarity current flowing through the diodes due to the induced reverse(d) polarity voltage on the capacitor C 110. As the reverse(d) polarity current may be higher than 10 A and may even exceed 25 A, which is approximately the maximal current rating of common off-the-shelf (OTS) diodes, the resistance R 150 must be sufficiently high, for example, on the order of several hundred to several thousand Ohms (Ω), in order to limit any reverse(d) polarity current to the maximum sustainable by common semiconductors.

This results in a serious drawback. Significant losses are induced as energy is dissipated in the resistor R 150 and diodes, wherein the power dissipated in a single sine cycle of the resonant oscillations can be in a range of hundreds of watts to kilowatts. Another serious drawback of having to include the resistor R 150 is that it prolongs the capacitor charging time and therefore reduces the maximal achievable magnetic pulse repetition rate.

A possible approach to address some of these drawbacks is presented in US 11,484,72 B2. As summarized in Fig. 2 of the present application, in a circuit 200 as suggested therein, the switch SW 160 is not connected in series but in parallel to the electromagnetic coil L 120. The resulting LC circuit does not require a resistor such as resistor R 150 and therefore avoids the dissipation losses associated therewith. However, the circuit 200 requires a power supply PS 130 capable of delivering high voltages in the range of 100 to 3,000 volts of direct current (Vdc) and must additionally be capable of withstanding continuously arising short circuit conditions which occur during each resonant oscillation of the LC circuit. In order to prevent short circuit conditions at the power supply PS 130, a resonant LLC topology may typically be used for the power supply PS 130, where short circuit conditions put the power supply PS 130 out of resonance. However, this is disadvantageous in that it makes the power conversion less efficient.

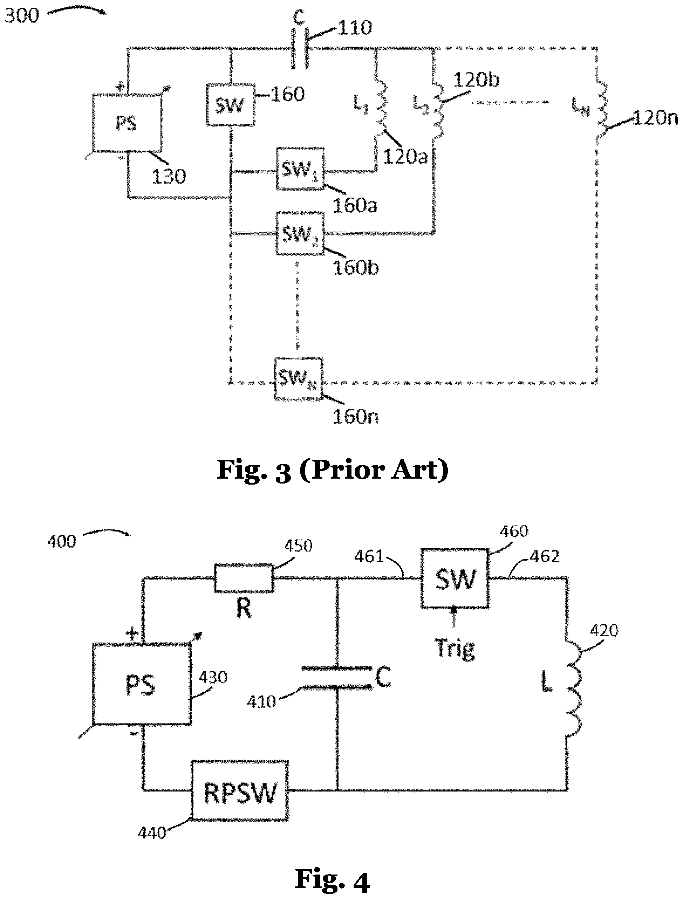

Another very important disadvantage of the resistor-free prior art devices as disclosed in US 11,484,72 B2, especially when considering therapies targeting large body areas, is that it is not possible, or at least cumbersome and/or inefficient, to switch among multiple coils L₁ 120a through L_{N} 120n (so-called multichannel operation) while using only a single capacitor C 110. As shown in Fig. 3, for multiple channels to be functional, additional switches SW₁ 160a through SW_{N} 160n have to be connected in series with the primary switch SW 160, wherein each of the additional switches corresponds to (controls) one (i.e., an individual one) of the multiple channels.

Consequently, each magnetic pulse requires synchronous activation of two switches in series. However, the series connection of switches introduces additional losses. For example, a typical voltage drops across an semiconductor controlled rectifier (SCR) type switch is equal to at least 2 V. Since the switching currents are in the range of 100 to 2000 A, already the activation of a single switch introduces a loss in the range of tens to several hundreds of watts. As there are always two switches that need to be activated, these losses double during each activation.

Therefore, there is a need for an improved circuit for generating a time-varying magnetic field for magnetic stimulation, addressing at least some of the above-described disadvantages of the prior art and further improving other aspects.

### Summary of the invention

A first aspect of the present invention relates to a circuit for generating a time-varying magnetic field for magnetic stimulation. The circuit comprises a capacitor bank comprising at least one capacitor, and a first electromagnetic coil. The capacitor bank and the first electromagnetic coil form a first LC circuit. The circuit further comprises a power supply for charging the capacitor bank by applying a charging voltage to the capacitor bank. In addition, the circuit comprises a switch configured to electrically disconnect the first LC circuit from the power supply when a voltage across the capacitor bank is reverse in polarity to the charging voltage.

The capacitor bank and the first electromagnetic coil forming a LC circuit act as a resonator, as described above. For example, a connection between the capacitor bank and the first electromagnetic coil may allow that a voltage across the capacitor can drive a current through the electromagnetic coil, which may in turn generate a time-varying magnetic field. Generally, the capacitor bank and the first electromagnetic coil may be connected in parallel to the power supply.

The power supply may be a direct voltage power supply. For example, the charging voltage may be applied such that the capacitor bank generates an electric field. Specifically, the electric field may be generated between plates of one or more capacitors of the capacitor bank. The power supply may have a positive and a negative pole. For example, the power supply may be configured as a current source while charging the capacitor bank and else as a voltage source maintaining a voltage across its poles. The voltage between the positive and the negative pole may define a polarity of the power supply and/or of the charging voltage.

In particular, the circuit comprises a switch which is configured to electrically disconnect the first LC circuit from the power supply when a voltage across the capacitor bank is reverse in polarity to the charging voltage. A voltage across the capacitor bank may be reverse in polarity to the charging voltage if the polarity of (the voltage across) the capacitor bank is opposite to the polarity of (the voltage across, e.g., the poles of) the power supply.

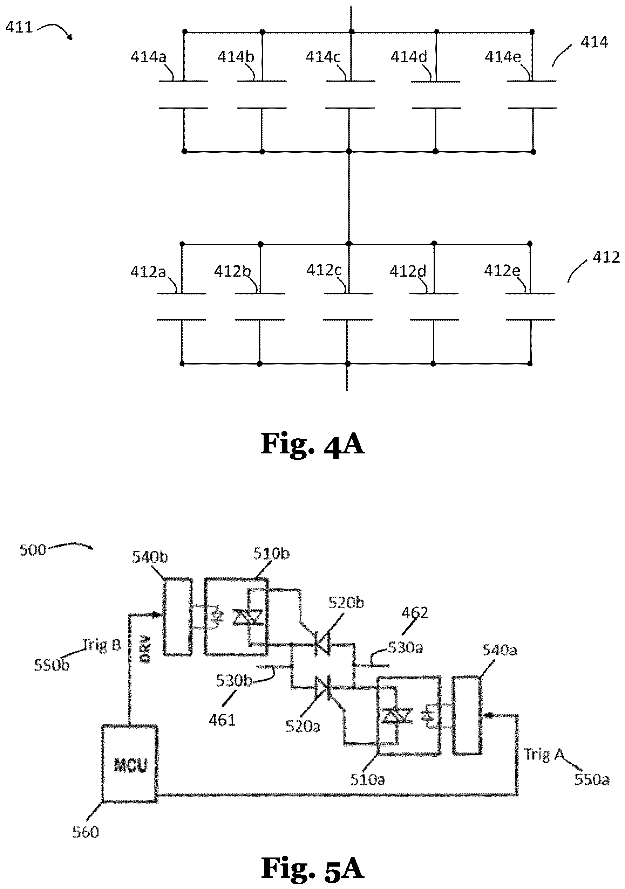

Electrically disconnecting may not necessarily comprise physically disconnecting the first LC circuit from the power supply. More generally, throughout this disclosure, a physical connection (e.g., between components) may not necessarily imply an electrical connection (between the components). For example, two components may be connected to/via a switch, but they may only be electrically connected when the switch is closed and electrically disconnected when the switch is open. It is to be appreciated that depending on the configuration of a switch the switch may be open when inactive/deactivated/not triggered (terms used interchangeably herein) and closed when active/activated/triggered (terms used interchangeably herein). This applies to electrically connecting and discontenting the first switch SW shown in Figs. 1 and 4 (discussed in more detail below). Alternatively, a switch may be closed when inactive/deactivated/not triggered, and open when active/activated/triggered. This may apply to electrically connecting and discontenting the switch RPSW as shown in Fig. 4 (discussed in more detail below).

According to the present invention, the first LC circuit is electrically disconnected from the power supply at least for a part of a duration during which a voltage of reverse polarity is present across the capacitor bank. That is, even though the switch is configured to electrically disconnect the first LC circuit from the power supply when a voltage across the capacitor bank is reverse in polarity to the charging voltage, there may be times during which a voltage of reverse polarity is present across the capacitor bank and during which the LC circuit is nevertheless not (yet) electrically disconnected from the power supply. This may, e.g., be due to delays/latencies in (de-)activating the switch. Additionally or alternatively, the first LC circuit may be electrically disconnected from the power supply whenever, e.g., at all times during which, a voltage of reverse polarity is present across the capacitor bank; e.g., the switch may be configured to electrically disconnect the first LC circuit from the power supply if, and, optionally, only if, a voltage across the capacitor bank is reverse in polarity to the charging voltage. In addition, or alternatively, electrically disconnecting the first LC circuit from the power supply when a voltage across the capacitor bank is reverse in polarity to the charging voltage may further comprise disconnecting the first LC circuit from the power supply over a period of time that comprises, but does not necessarily consist exclusively of, times at which a reverse polarity is present across the capacitor bank. In some embodiments, the first LC circuit and the power supply may be electrically disconnected when a voltage of reverse polarity across the capacitor is detected, and the disconnection may persist although the polarity across the capacitor bank may, at a later point in time, coincide again with the polarity of the power supply. For example, the first LC circuit and the power supply may be/remain electrically disconnected for a period of time comprising at least one oscillation of the LC circuit.

Electrically disconnecting the LC circuit from the power supply when a voltage across the capacitor bank is reverse in polarity to the charging voltage protects the power supply, particularly the diodes of the power supply, from the reverse polarity voltage across the capacitor bank. In particular, as the protection is not based on a resistor adapted to limit the reverse current flowing through the diodes of the power supply, the circuit according to the present invention avoids the significant losses the state of the art entails. At the same time, as will become apparent more clearly below, the present invention avoids series connections of two switches in multichannel embodiments, also in this regard avoiding the significant losses the state of the art entails. Additionally, in preferred embodiments of the present invention, the levels of electromagnetic emissions may be further minimized, which is of particular advantage for a magnetic stimulation apparatus for which maximally allowed levels of electromagnetic emissions are limited by stringent standards and regulations for medical devices.

Overall, the circuit according to the present invention hence provides for particularly efficient means to generate a time-varying magnetic field for magnetic stimulation, avoiding the drawbacks associated with the above-discussed state of the art.

In general, the circuit may comprise a resistance. Preferably, the resistance may be integrated into the power supply. The resistance may be configured to protect the power supply when a voltage across the capacitor bank is reverse in polarity to the charging voltage. In particular, the resistance may protect the power supply in case the switch electrically disconnects the first LC circuit and/or capacitor bank from the power supply with a delay/latency. For example, the resistor may protect the power supply when a voltage across the capacitor bank is reverse in polarity to the charging voltage, but the switch has not (yet) electrically disconnected the first LC circuit and/or the capacitor bank from the power supply. In other words, including a resistance may be beneficial as it allows reducing a sensitivity of the circuit to a miss-timing and/or an incorrect disconnecting of the switch. For example, an incorrect disconnecting may occur when the reverse polarity is within a vicinity of a reference value of the switch.

In particular, the resistance may be smaller than 100 Ω, more preferably smaller than 50 Ω, even more preferably smaller than 15 Ω, most preferably smaller than 10 Ω. Using such a resistance may allow to easily integrate the resistance into the power supply. As such, the resistance may be significantly smaller as those used in the state of the art (cf. Fig. 1) to protect the power supply from reverse(d) polarity. Further, by using such a resistance, the power dissipated in the resistor is significantly reduced, thereby minimizing overall power dissipation/consumption of the circuit. In addition, as less heat is dissipated/generated, the heating of the electrical components of the circuit is reduced. As heating of the electrical components may result in a need for cooling and/or in a downtime of the circuit, the operating time of the circuit is increased, which further optimizes the treatment of a patient.

Specifically, the resistance and the switch may be connected in series to the power supply. Specifically, the switch may be connected in series to the power supply, within a branch connected to a negative pole of the power supply or within a branch connected to a positive pole of the power supply. Similarly, the resistance may be connected in series to the power supply, within a branch connected to the negative pole of the power supply or within a branch connected to the positive pole of the power supply. In addition, or alternatively, the resistance may separate the first LC circuit from the switch.

Preferably, the switch may be connected in series to the negative pole of the power supply. By being connected to the negative pole of the power supply, the trigger circuit of the switch is not exposed to the full capacitor bank voltage at all times, thus avoiding high root mean square (RMS) voltage fluctuations. As a result, the electromagnetic interference (EMI) emissions are reduced, and the reliability of the switch's circuit is improved. Further, the maximum repetitive peak isolation voltage specification requirements for the isolation components of the switch are also minimized.

Alternatively, the resistance may be connected in series to the positive pole of the power supply since the positive pole is always impedance connected with lowest impedance towards a protective earthing (PE) through an impedance of the circuit. Generally, an RC network (as will be described in more detail below) resistance lowers dU/dt transients and therefore reduces EMI emissions, thereby improving the performance and reliability of the circuit.

The resistance may also be connected in series to the negative pole of the power supply. For example, the negative pole of power supply may comprise (e.g., be connected to) the switch, e.g., the RPSW switch of Fig. 4 (discussed in more detail below), which disconnects the negative pole of the power supply from the capacitor bank in high-impedance state, during a magnetic pulse, e.g., for a duration from π/4 to 3π/4, when a voltage across the capacitor bank is negative. As a result, the negative pole of the power supply will have high dU/dt transients, in turn yielding increased EMI emissions. Thus, in some embodiments, the resistance is preferably connected in series to the positive pole of power supply.

Therefore, in a most preferred embodiment, the switch may be connected in series to the negative pole of the power supply, and the resistance may be connected to the positive pole of the power supply. This results in the minimization of electromagnetic emissions and further improves the reliability of the circuit. This is especially important since there are stringent regulatory requirements for electromagnetic emissions for medical devices. However, in other embodiments, the switch and the resistance may each be connected either to the positive or negative pole of the power supply.

Generally, the capacitor bank may comprise between 1 capacitor up to 20 capacitors. When a capacitor bank comprises only a single capacitor, that capacitor may have to be large in volume and size, making the apparatus undesirably large. Further, a single capacitor may have to sustain large currents leading to substantial heating up of the capacitor's electrical connections, and of the capacitor itself, due to the small surface-to-volume ratio of the capacitor. This temperature stress may also lead to premature capacitor failure due to expansion of materials at higher temperatures.

For reduced internal resistance with improved efficiency and reduced heating the capacitor bank may comprise at least one capacitor set comprising at least two, and up to 20 capacitors connected in parallel.

Further, in case the voltage rating of the capacitors is lower than the charging voltage of the power supply the capacitor bank may comprise at least two capacitor sets connected in series, wherein each of the sets comprises at least two and up to 10 or even 20 capacitors connected in parallel.

In one of the preferred embodiments, the capacitor bank may comprise two sets connected in series, each set comprising five capacitors connected in parallel. Such configurations/embodiments of the capacitor bank have been found to result in a low internal resistance. By reducing internal resistance, the capacitor bank operates more efficiently, resulting in improved energy transfer and reduced power losses. Furthermore, reducing the internal resistance minimizes the need for cooling the capacitor bank. In addition, the lifetime of the capacitors in the capacitor bank is extended due to due total resonant current being split across several parallel connections. In general, the circuit offers flexibility in the configuration of the capacitor bank, allowing for customization based on specific application requirements.

In general, the circuit may further comprise a first switch for electrically closing and opening the first LC circuit. Generally, the first switch (and, optionally, also the first electromagnetic coil) may be connected in series to the power supply.

Similar to what has been explained above already, electrically closing and opening a connection may not necessarily comprise a mechanical closing and opening of the connection. Closing the first LC circuit by means of the first switch may comprise establishing an electrical connection between the first electromagnetic coil and the capacitor bank. In particular, closing the first LC circuit by means of the first switch may allow the capacitor bank to be discharged (into the first electromagnetic coil). Conversely, opening the first LC circuit by means of the first switch may comprise electrically disconnecting the first electromagnetic coil from the capacitor bank and/or the power supply. For example, opening the first LC circuit by means of the first switch may allow to charge at least a part of the capacitor bank by applying the charging voltage of the power supply. The first switch and the resistance may be connected in series to the power supply.

Furthermore, the circuit may be configured to trigger the first switch based on a plurality of pulses. The plurality of pulses may comprise a first pulse adapted to set a forward connection of the first switch to a conductive state and a second pulse adapted to set a reverse connection of the first switch to a conductive state, wherein the second pulse is generated after the first pulse but before a current through the first switch reverses (its) direction.

Triggering the first swich, in particular its forward and reverse connections, based on a plurality of pulses may allow to electrically open and/or to electrically close the first LC circuit by means of the first switch. This allows for particularly precise control and in turn a particularly reliable operation of the circuit as will be explained in the following.

For example, a first pulse may be adapted to set a forward connection of the first switch to a conductive state. Setting a forward connection into a conductive state may comprise that the connection may conduct a current in a forward direction. A forward direction may be a direction of a current in the circuit induced (solely) by the polarity of the power supply, e.g., during charging of the capacitor bank. In some embodiments, a forward connection which is not in a conductive state may not conduct a current with respect to the forward direction.

Similarly, setting a reverse connection to a conductive state may comprise that the connection may conduct a current in a reverse direction. A reverse direction may refer to a direction reverse with respect to a forward direction. For example, a reverse direction may be a direction of a current which is opposite to a direction of a current induced (solely) by the polarity of the power supply, e.g., during charging of the capacitor bank.

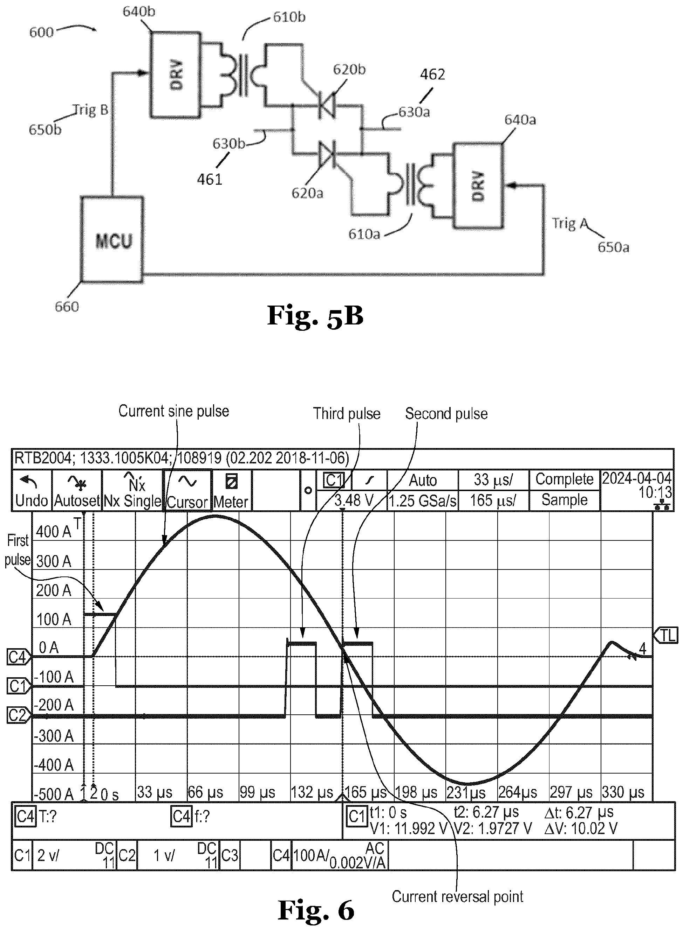

In general, the second pulse may be generated after the first pulse but before the current through the first switch falls below zero. In general, the forward connection may remain in a conductive state as long as the current through the first switch remains positive, i.e., as long as the current through the first switch does not reverse (its) direction. Therefore, an optimal timing of the second pulse may be such that a reverse connection of the first switch is set to a conductive state at time tᵣ/2, i.e., when the current through the first switch reaches zero (e.g., a zero value) and reverses (its) direction, as will be discussed in more detail below with reference to Fig. 6.

The plurality of pulses may further comprise a third pulse adapted to set the reverse connection of the first switch to the conductive state. Specifically, the third pulse may be adapted to ensure that the reverse connection of the first switch is reliably set to the conductive state.

The third pulse may be generated before the second pulse, its role being to shorten the opening time of the first switch in reverse direction when the second pulse arrives.

In some embodiments, there may be further pulses similar to the third pulse, each adapted to set the reverse connection of the first switch to the conductive state, with all pulses adapted to set the reverse connection of the first switch to the conductive state (immediately) following one another.

For example, the second pulse may fail to set the reverse connection to a conductive state before the current through the first switch reverses (its) direction. In this case, the third pulse may ensure that the reverse connection of the first switch is timely set to the conductive state. Thus, employing (at least) three pulses in total, (at least) two adapted to set a reverse connection of the first switch to a conductive state, allows for a more secure triggering of the first switch, in particular a more secure change from a conductive forward connection to a conductive reverse connection. In particular, using a third pulse minimizes the possibility that the reverse connection is not in a conductive state when the current through the first switch reverses direction, thus minimizing the risk of harming electronic components of the circuit. Further, the possibility of mistriggering of the first switch is minimized.

In general, the first pulse, the second pulse and/or the third pulse (and any further pulse similar to the third pulse) may have a respective duration which may be based at least in part on a resonant frequency fᵣ and/or a resonant period tᵣ of the first LC circuit. In particular, the durations may depend on the capacitance of the capacitor bank and/or the inductance of the first electromagnetic coil. The first pulse and/or the second pulse and/or the third pulse may have the same duration of about 0.05 tᵣ to 0.15 tᵣ. The second pulse may be generated at times 0.015 tᵣ to 0.17 tᵣ before the half period of the current's sine oscillation, i.e., before the current through the first switch reverses direction. In addition, or alternatively, the third pulse may be generated at times 0.07 tᵣ - 0.25 tᵣ before the current through the first switch reverses direction. In addition, or alternatively, the second pulse and the third pulse have a mutual distance of 0.05 tᵣ - 0.15 tᵣ.

The inventors have found that these respective durations and mutual distances provide for particularly good results; specifically, they provide a particularly large degree of certainty that the first switch is not mis-triggered.

The first switch may comprise a bipolar connection of at least two thyristors, in particular semiconductor-controlled rectifiers, SCRs, one of the at least two thyristors forming the forward connection and another of the at least two thyristors forming the reverse connection. More generally, the switch may be a semiconductor switch such as a SCR, a metal oxide semiconductor field-effect transistor (MOS-FET), a gallium nitride field-effect transistor (FAN-FET), a silicon carbide field-effect transistor (SIC-FET) and/or an insulated-gate bipolar transistor (IGBT) connected in a bipolar configuration.

The switches may be configured to switch high voltages in the range of 300 V to 4000 V. In some embodiments, the electric current switching capability may be increased by a parallel operation of semiconductor switches which may enable driving currents in the range of 100 to 2000 A. In addition, or alternatively, the voltage rating of the (semiconductor) switch may be increased by a series connection of individual (semiconductor) switches. The voltage rating and/or the current rating of the switch may depend on the magnetic stimulation therapy to be applied.

In general, at least one of the plurality of pulses (i.e., at least one of the plurality of triggering pulses for the first switch) may be generated by a trigger transformer and/or a phototriac optocoupler. Generating at least one of the plurality of pulses by a trigger transformer and/or a phototriac optocoupler may allow to set a forward connection of the first switch to a conductive state and/or to set a reverse connection of the first switch to a conductive state. In some embodiments, the whole plurality of pulses is either generated by a trigger transformer or a phototriac optocoupler. In general, the trigger transformer and/or a phototriac optocoupler may be part of a trigger circuit.

The trigger circuit may comprise a microcontroller, which may be configured to generate at least one low voltage digital control pulse. The first pulse and/or the second pulse and/or the third pulse may be based at least in part on the low voltage digital control pulses. The inventors have found such embodiments, especially such using a trigger transformer and/or a phototriac optocoupler, to be particularly reliable.

In general, the circuit must be configured such that a triggering of the switch, resulting in disconnection of the power supply from the LC, may occur before or latest at the time of the reversal of the capacitor voltage which occurs at a quarter period of the current sine pulse through the first switch. Similarly, a second triggering of the switch, which may reactivate the connection between the power supply and LC circuit, may occur after the voltage re-reverses polarity and becomes positive again, i.e., after 3/4 tᵣ.

Optimally, in order to allow optimal duration of a recharging of the capacitor bank by the voltage of the power supply, the switch should be triggered such that it remains open only during the time when the voltage is negative. In general, the switch may be triggered based on (e.g., the switch may comprise) a comparator circuit configured to detect a voltage reverse in polarity to the charging voltage. Triggering the switch based on a comparator circuit allows for a comparatively simple design and/or implementation of the circuit, potentially enhancing reliability and reducing production costs.

The switch and the first switch may be also triggered such that the switch electrically disconnects the first LC circuit from the power supply when the first switch electrically closes the first LC circuit. Triggering the switch and the first switch such that the switch electrically disconnects the first LC circuit from the power supply when the first switch electrically closes the first LC circuit may protect the power supply from a reverse polarity voltage across the capacitor bank. Synchronously triggering the switch and the first switch may comprise that the switch and the first switch are triggered by, or at least based on, a same pulse. For example, the pulse for triggering the switch may be based on and/or generated from the same controller for triggering the first switch. In particular, the switch may be triggered latest when the voltage across the capacitor bank reverses its polarity, e.g., at tᵣ/4.

In some embodiments, the circuit may comprise a second electromagnetic coil, such that the capacitor bank and the second electromagnetic coil form a second LC circuit. Optionally, the circuit may comprise a second switch for electrically closing and opening the second LC circuit. Generally, the second switch may be further configured to electrically disconnect the second LC circuit from the capacitor bank when the current through the second switch reverses its direction, e.g., at tᵣ/2.

In particular, the second electromagnetic coil may be connected in parallel with the capacitor bank through the second switch, and in parallel with the power supply through the switch.

In general, the inductance of the second electromagnetic coil may be different from, or the same as, the inductance of the first electromagnetic coil.

The second switch may be connected to the capacitor bank in series with the second electromagnetic coil.

The first switch and the second switch may be a same type of switch and/or may have same physical properties. In general, the properties of the second switch may depend on the second electromagnetic coil and/or the second LC circuit. In some embodiments, the first switch and the second switch may be opened and/or closed in a pattern. For example, the first switch and the second switch may be opened and/or closed in an alternating pattern, meaning that when the first switch is open, the second switch is closed and/or when the first switch is closed, the second switch is open.

Having a second electromagnetic coil and/or a second LC circuit may allow for a multichannel configuration (e.g., the use of multiple applicators substantially simultaneously) of the circuit/of a magnetic stimulation apparatus using a circuit according to the present invention. That is, multiple electromagnetic fields may be generated by different electromagnetic coils, which may form part of respective different applicators. This allows for performing therapy over a large body area, as designing and powering a very large area single coil applicator is technically challenging. In particular, magnetic fields generated by different magnetic coils may cover different body areas of the patient. In general, the different electromagnetic coils may be consecutively switched on and off, e.g., at a switching rate.

As compared to multichannel configuration known from the state of the art (cf. Fig. 3), the losses associated with the switches may be substantially reduced using a multichannel configuration according to the present invention.

In general, the circuit may comprise more than two electromagnetic coils (up to i =N), preferably wherein, for each additional electromagnetic coil, the circuit further comprises an additional, corresponding i^{th} switch. For example, the circuit may comprise four electromagnetic coils and four corresponding first through fourth switches, next to the switch configured to electrically disconnect any of the resulting LC circuits from the power supply when a voltage across the capacitor bank is reverse in polarity to the charging voltage.

According to present invention, the circuit may further comprise an RC network. The RC network may be used to reduce working voltages on safety isolating barriers inside the medical equipment that is, or may comprise, aspects of the present invention, in particular a circuit according to the present invention. Basic medical standards prohibit the presence of mains voltages, e.g., voltages derived from primary circuits in galvanic connection with the mains, on a patient.

Thus, to provide suitable isolation of the patient, safety barriers need to be provided between mains parts and patient and/or operator, e.g., a minimum of two means of patient protection for working voltages occurring on safety isolation barrier MAINS - PATIENT and, e.g., a minimum two means of operator protection for working voltages occurring on safety isolation barrier MAINS - OPERATOR. By means of present invention or implementation of RC network, the working voltage occurring on isolation barrier MAINS - PATIENT and/or MAINS - OPERATOR, is reduced by almost factor of two. Basic medical standards define quite strict requirements concerning clearance and creepage to cover also worst-case scenarios. Therefore, it is of great importance to achieve sufficiently low working voltages on all safety isolating barriers, which ensures standards compliance and results in better equipment performance and reduced creepage and clearance distances, which significantly reduce size of the equipment.

The RC network may fix a floating potential of the (entire) first LC circuit and/or second LC circuit and/or any further LC circuit to the protective earthing (PE) potential. This solution may nearly halve the working voltage between primary mains circuits and patient circuits and between patient circuits and secondary circuits isolated from mains by reinforced isolation or two means of operator and/or patient protection.

Further, the connection of all comprised LC resonant circuits may be electrically connected to ground via a protective earthing (PE) node, wherein the connection to PE protective earthing comprises at least one RC pair of an RC network, wherein RC network is comprised of one or more RC pairs. Each RC pair may be symmetrically connected on each side of the PE protective earthing node, wherein an upper RC pair may be connected to an upper leg of the capacitor bank and a lower RC pair may be connected to a lower leg of the capacitor bank. Each RC pair is typically comprised of a parallel connection of a capacitor and a resistor. A symmetrical connection of two or more RC pairs may be used to form an RC network, wherein a middle point of the RC network is connected to the PE protective earth.

Using the same number of pairs on each side of the PE node, in particular wherein the secondary resistances and/or the secondary capacitances of the RC pairs coincide in value, may minimize a working voltage of safety barriers, resulting in a small creepage and clearance distances and therefore optimizing the performance of the circuit, as well as its suitability, for medical treatments.

Generally, the capacitor bank may be configured such that an airflow can be formed, wherein the airflow is adapted to cool at least a part of the capacitor bank. The circuit may further comprise a temperature sensor configured to measure the temperature of at least a part of the capacitor bank.

Configuring the capacitor bank such that an airflow can be formed may allow for particularly efficient cooling and temperature management of the capacitor bank. In particular, the capacitor bank and/or the capacitors comprised in the capacitor bank may be positioned such as to facilitate an efficient airflow around each individual component of the capacitor bank and/or the circuit. In general, the airflow cooling mechanism may help dissipate heat generated during operation, thus preventing overheating and ensuring the longevity of the capacitor bank. In addition, or alternatively, the improved cooling design may allow for the use of a capacitor bank and/or capacitors with higher power ratings, further enhancing the circuit's performance and reliability. A temperature sensor may be used to regulate the cooling power. In addition, or alternatively, the temperature sensor may be configured to limit or stop the operation of the circuit.

As already pointed out, the circuit according to present invention is particularly adapted to achieve high magnetic pulse rates (e.g., pulse repetition rates) and at the same time to produce strong magnetic fields, rendering intense nerve stimulation at high pulse repetition rates possible. Furthermore, especially also using multiple channels, the circuit may be used to cover a large body area with a time-varying magnetic field.

The inventors have found that this makes the circuit according to the present invention particularly well-suited for reducing inflammation in a patient, as will become clear from the following. Consequently, a second aspect of the present invention relates to a method for reducing inflammation in a patient. The method comprises applying a time-varying magnetic field to at least one muscle of the patient to magnetically stimulate the at least one muscle, wherein the magnetic field is adapted such as to trigger at least one contraction of the at least one muscle.

It is generally known that (regular) physical activity, combining the activation of myokines and promoting the reduction of visceral fat, helps control inflammation, also the kind of low-grade chronic inflammation that has become known as inflammaging - a process that increases the risk of chronic degenerative illnesses and decreases life quality in the adult aging population. A chronic state of inflammation entails many, particularly health-related, problems, e.g., persistent fatigue, body discomfort including joint stiffness, tendonitis and muscle pain, gastrointestinal complications such as diarrhea or constipation, weight gain, unexplained weight loss, skin rashes, persistent infections and symptoms of mood disorders, including depression and anxiety - all in a range from mild to severe, lasting for several months or years. Also, a chronic state of inflammation leads to hyperinsulinemia, which in turn promotes insulin resistance with the increased production of IGF-1 (insulin-like Growth Factor-1) and hormone imbalances. That said, a chronic state of inflammation may also well be present in persons with normal weight and without insulin resistance, since inflammation can be derived from other ailments such as adrenal stress, psychological stress, metal toxicity, electromagnetic smog and others.

While other approaches to managing chronic inflammation are available (e.g., drugs of different kinds), these other approaches are generally less effective and/or entail severe side effects. For example, a long-term use of nonsteroidal anti-inflammatory drug has been associated with the risk of heart attack and/or stroke. Further, steroids may have significant side effects like weight gain, bone thinning and/or mood swings. Meanwhile, a regime of (regular) physical activity is often hard to implement in practice. The biggest obstacle remains in the patient's willingness to adopt and adhere to them, especially when considering the need to sustain them for prolonged periods of time during which the effects are not immediately perceived.

The present invention addresses this issue. As per the basic principle of magnetic stimulation, a time-varying magnetic field may induce an electrical current in motor neurons which may result in an involuntary muscle contraction. Involuntary muscle contraction may in turn contribute to anaerobic exercise. While most studies on the effects of muscle exercise on the release of myokines have arguably focused on aerobic exercise (i.e., on cardiovascular exercise during which the patient's heart starts pumping), it has been found that anaerobic exercise through externally stimulated involuntary muscle contraction can exert positive effects on the release of various myokines, too, as has been demonstrated by electrically stimulated contractions of rat muscles.

Notably, as anaerobic exercise may be less effective in reducing inflammation than aerobic exercise, for efficient anaerobic treatment a large enough muscle area needs to be stimulated. Consequently, using electrical stimulation is not suitable for controlling inflammation, as electric stimulation requires direct skin contact. In particular, electrical stimulation would require an unpractically large direct skin contact area that would be very uncomfortable for the patient at the required electrical stimulation intensities. Therefore, using a time-varying magnetic field is advantageous over electrical stimulation as no direct skin contact is required. In fact, the time-varying magnetic field can pass any medium, even a vacuum, without attenuation of energy and thereby provides the patient with a powerful, effective, comfortable, non-invasive, motivating, painless fast and safe method to obtain high muscle contraction activity. In particular, as no directs skin contact is needed, magnetic stimulation therapy is also accessible for patients with extreme hypersensitivity or allodynia to skin touch.

Reducing inflammation, particularly low- and/or medium-grade chronic inflammation by stimulating at least one muscle with a time-varying magnetic field does not entail any of these drawbacks. It is advantageous as no unwanted and/or significant side effects are caused. Further, reducing inflammation, particularly low- and/or medium-grade chronic inflammation by stimulating at least one muscle with a time-varying magnetic field is advantageous as it minimizes and/or eliminates the common symptoms of chronic inflammation. In particular, it may minimize and/or eliminate persistent fatigue, body discomfort including joint stiffness, tendonitis and muscle pain, depression or anxiety, gastrointestinal complications, e.g., diarrhoea or constipation, weight gain, unexplained weight loss, skin rashes, persistent infections and mood disorders including depression and anxiety.

In general, the time-varying magnetic field may at least in part be based on an electromagnetic coil. For example, at least a part of the time-varying magnetic field may be generated by the above-described circuit according to the first aspect of the present invention. In particular, at least one electromagnetic coil of the circuit may generate a time-varying magnetic field stimulating the at least one muscle of the patient. However, the second aspect of the present invention is not limited thereto; a method for reducing inflammation in a patient according to the second aspect of the present invention may equally well use any other circuit for generating the time-varying magnetic field.

The time-varying magnetic field and/or the applicator comprising the respective electromagnetic coil may be positioned at a distance from the at least one muscle or muscle group, wherein the distance may depend on the muscle type, the obesity of the patient, preferably characterized by the body weight index (BMI), and/or the sex of the patient. Generally, the distance may be defined as a distance to a half-thickness of the muscle or muscle group. The half-thickness of a muscle or muscle group may be a thickness at one particular location of the muscle or muscle group, for example, the location where the thickness of the muscle is maximal or minimal. In addition, or alternatively, the thickness of a muscle or muscle group may be an average thickness or any function of an elongation of the muscle or muscle group.

Generally, an application of the time-varying magnetic field may be adapted to trigger and/or enhance myokine production in the patient. For example, triggering and/or enhancing myokine production in the patient may be based on triggering at least one deep penetrating muscle contraction.

In general, a muscle is a dynamic tissue as well as an endocrine organ that produces proteins that regulate metabolism and also has other important functions. Muscle contraction, also when induced by a time-varying magnetic field, may activate skeletal muscle fibres and thereby produce and release several proteins. These proteins may comprise myokines, for example, myostatin, musclin, irisin, interleukin-6 (IL-6) and/or interleukin-1 (IL-1). The myokines may be released into the bloodstream and may exert respective effects in an autocrine, a paracrine and/or endocrine manner. At least a part of these effects may create affects in different organs, for example a brain, adipose tissue, bone, liver, gut, pancreas and/or vascular bed and/or skin. An increase in IL-6 may stimulate an anti-inflammatory systemic environment. Therefore, IL-6 promotes an increase in the production of other anti-inflammatory cytokines.

In particular, the method may be used for reducing low- and/or medium-grade chronic inflammation. For example, the myokine production triggered by the time-varying magnetic field may be used for managing chronic systematic inflammation. Managing chronic systematic inflammation may comprise reducing a level of insulin and/or of a tumor necrosis factor (TNF), preferably TNF-*α*. In particular, managing chronic systematic inflammation may be used for reducing low- and/or medium-grade chronic inflammation.

The magnetic field may be adapted such as to trigger at least one contraction of at least one large muscle or muscle group of the patient exceeding 300 cm³. For example, the at least one large muscle may include a gluteal muscle and/or a quadriceps of the patient.

Triggering at least one contraction of at least one large muscle or muscle group of the patient exceeding 300 cm³ may comprise that at least an area of 300 cm³ of the muscle or the muscle group is contracted. For example, the large muscle or the muscle group exceeding 300 cm³ may comprise a coherent and/or connected area of the patient. In some embodiments, the area of at least 300 cm³ may be distributed across at least two unconnected muscles and/or areas of the patient.

Contraction of at least one large muscle or muscle group of the patient exceeding 300 cm³ is advantageous because the production of anti-inflammatory myokines depends on the amount of contracted muscle mass and is therefore strongly related to the amount of muscle mass exercised/contracted. Therefore, in order to maximize the production of myokines, large muscle groups, such as those located in the arms, thighs, abdomen and buttocks, may be targeted.

In general, triggering a contraction of at least one large muscle or muscle group by a time-varying magnetic field may be based at least in part on the circuit according to the first aspect of the present invention. For example, the circuit may comprise a plurality of electromagnetic coils. In particular, the different electromagnetic coils, potentially arranged in a same applicator or different applicators, may be arranged such as to stimulate and/or to treat different muscles and/or muscle groups. For example, the time-varying magnetic field of the different electromagnetic coils may stimulate and/or treat different muscles and/or muscle groups jointly, preferably covering areas of at least 200 cm², more preferably of at least 1000 cm², even more preferably at least 2,000 cm², most preferably at least 3,000 cm².

Specifically, triggering a contraction of at least one large muscle or muscle group of the patient may be at least in part based on the multichannel embodiment of the above-described circuit. In particular, for a substantially simultaneous stimulation a plurality of electromagnetic coils may be placed over different body areas and/or corresponding muscles. For example, the first electromagnetic coil of the first LC circuit may be placed over a first body area and/or corresponding muscle and a second electromagnetic coil of a second LC circuit may be placed over a second body area and/or corresponding muscle.

Accordingly, the method may comprise magnetically stimulating, sequentially and/or (substantially) in parallel, at least two muscles of the patient, wherein the two muscles preferably belong to different muscle groups. For example, a first muscle may belong to the gluteus muscles and a second muscle to the quadriceps. In some embodiments, the at least two muscles may belong to the same muscle group. For example, the first and the second muscle may belong to the gluteus muscles, including gluteus maximus, gluteus medius and/or gluteus minimus. This allows to stimulate, in total, a large muscle mass, thereby optimizing the production of anti-inflammatory myokines.

In general, a treatment session may last at least 25 minutes, preferably at least 35 minutes, most preferably at least 45 minutes, and/or at most 120 minutes, preferably at most 90 minutes, most preferably at most 60 minutes. The inventors have found that treatment results may be optimal under these circumstances. A treatment session of at least 25 minutes, preferably at least 35 minutes, most preferably at least 45 minutes guarantees that a sufficient amount of anti-inflammatory myokines is produced and thereby inflammation, particularly low- and/or medium-grade chronic inflammation is reduced. At the same time, a treatment session of at most 120 minutes, preferably at most 90 minutes, most preferably at most 60 minutes may prevent soreness of the muscles and/or muscle groups of the patient.

A treatment session may comprise treating one muscle and/or muscle group during the treatment session. In addition, or alternatively, a treatment session may comprise the treatment of different muscles and/or muscle groups. For example, in a first part of a treatment session a first muscle and/or muscle group may be treated, and in a second part of the treatment session a second muscle and/or muscle group may be treated. Generally, the first muscle and/or muscle group may be different from the second muscle and/or muscle group. In some embodiments, the first muscle and/or muscle group may comprise the second muscle and/or muscle group. Alternatively, the first muscle and/or muscle group may be a subset of the second muscle and/or muscle group.

A duration of a first part of a treatment session may be similar and/or equal to a duration of a second part of the treatment session. For example, in a first part of the treatment session at least a part of the gluteal muscles may be treated and in a second part of the treatment session at least a part of the quadriceps may be treated. In particular, the first part may last at least 45 minutes and may comprise a treatment of the left and right glutes and the second part may last at least 45 minutes and may comprise a treatment of the left and right quadriceps. For example, the treatment may be based at least in part on the above-described circuit. In general, the second part of the treatment may be performed with the same circuit and/or applicator used in the first part of the treatment.

The method may further comprise magnetically stimulating the at least one muscle of the patient with varying magnetic field intensity, preferably varying peak intensity of a sequence of magnetic pulses, wherein the intensity preferably increases throughout a treatment session and/or throughout subsequent treatment sessions.

Varying magnetic field intensities may refer to a different time-dependency of the intensity of different magnetic fields. Time-dependency of the intensity may comprise a different gradient and/or a different time derivative. Furthermore, magnetic fields may vary in the peak intensity of the magnetic field. In particular, the magnetic field may vary in time-dependency of the intensity and peak intensity of the magnetic field. In general, the peak intensity and/or the time-dependency of the magnetic fields may depend on the treated muscle type, the body mass index and the sex of the patient. Furthermore, the intensity of the magnetic field may depend in the desired current which should be induced during the stimulation. For example, typical values of the induced currents may be at least 5 mA, preferably at least 7 mA, most preferably at least 10 mA. Alternatively, or in addition, the induced currents may be at most 200 mA, preferably at most 150 mA, most preferably at most 100 mA. In general, the induced current may depend on the muscle group, the body mass index and/or the sex of the patient. For example, the intensity of the magnetic field may be at least 50 Gauss, preferably at least 100 Gauss, more preferably at least 150 Gauss, most preferably at least 200 Gauss. In addition, or alternatively, the intensity of the magnetic field may be at most 50,000 Gauss, preferably at most 25,000 Gauss, more preferably at most 10,000 Gauss, most preferably at most 5,000 Gauss.

In general, different muscles and/or muscle groups may be treated with different magnetic field intensities. For example, the above-described circuit may be used for treating different muscle and/or muscle groups with different magnetic field intensities. For example, a first electromagnetic coil (and/or corresponding applicator) may be associated with a first intensity of a magnetic field and a second electromagnetic coil (and/or corresponding applicator) may be associated with a second intensity of the magnetic field.

The intensity of the magnetic field may increase throughout a treatment session and/or throughout subsequent/consecutive treatment sessions. For example, a treatment session may start with a low magnetic field intensity which then gradually increases throughout the treatment session and/or throughout subsequent/consecutive treatment sessions. In other embodiments, the magnetic field intensity may be essentially constant throughout a treatment session and/or throughout subsequent/consecutive treatment sessions.

Increasing the magnetic field intensity throughout a treatment session and/or throughout subsequent/consecutive treatment sessions contributes to maximizing the production of anti-inflammatory myokines, minimizing the inflammation in the patient, and thus optimizing the treatment.

Generally, the method may comprise magnetically stimulating the at least one muscle of the patient throughout multiple treatment sessions occurring at least once a week, preferably twice a week, for at least two weeks, preferably four weeks, most preferably six weeks.

Stimulating the at least one muscle of the patient throughout multiple treatment sessions may comprise stimulating a same at least one muscle of the patient in each of the multiple treatment sessions. In other embodiments, different muscles and/or muscle groups may be stimulated in different treatment sessions. For example, a first muscle and/or muscle group may be stimulated in a first treatment session and second muscle and/or muscle group may be stimulated in a second treatment session. In addition, or alternatively, the different muscles and/or muscle groups may alternate throughout multiple treatment sessions. For example, in a third treatment, a third muscle and/or muscle group may be stimulated, wherein the third muscle and/or muscle group coincide with the first muscle and/or muscle group, respectively.

Stimulating the at least one muscle of the patient throughout multiple treatment sessions occurring at least once a week for at least two weeks contributes to maximizing the production of anti-inflammatory myokines, minimizing the inflammation in the patient, and thus optimizing the treatment. Further, stimulating the at least one muscle at least once a week, preferably twice a week allows the at least one muscle to relax, thereby prohibiting soreness of the at least one muscle and thus optimizing the comfort of the patient throughout the treatment sessions.

In general, a body part of the patient comprising the at least one muscle may be covered at least partly by clothing during the method. As outlined earlier, a magnetic field can pass any medium, even a vacuum without attenuation of energy. Therefore, clothes of the patient do not hamper the magnetic stimulation of the at least one muscle and/or the treatment of the patient. Consequently, the patient does not need to undress during the treatment, thereby optimizing the comfort of the patient during the treatment.

Generally, the method may be performed based on (e.g., using) at least one applicator, comprising at least one electromagnetic coil. For example, the at least one applicator may be configured to apply a time-varying magnetic field to at least one muscle of the patient to magnetically stimulate the at least one muscle, wherein the magnetic field is adapted such as to trigger at least one contraction of the at least one muscle. The at least one applicator may be a hand-held device. In some embodiments, the at least one applicator may form at least a part of a chair, adapted to receive the patient in a sitting position.

In general, the at least one applicator may be at least partially based on the above-described inventive circuit for generating a time-varying magnetic field. In particular, the at least one applicator may comprise at least a part of the circuit. For example, the applicator may comprise the first and/or the second electromagnetic coil. The at least one applicator may in turn be connected, or connectable, to a main unit, which main unit may comprise at least a part, e.g., the remaining part, of the above-described inventive circuit for generating a time-varying magnetic field, such as the power source and/or the capacitor bank. Thus, the method may be performed based on (e.g., using) at least one applicator as well as such a main unit. More generally, the method may be performed based on (e.g., using) the above-described inventive circuit for generating a time-varying magnetic field.

Specifically, the method may be performed based on (e.g., using) a plurality of applicators and/or electromagnetic coils. For example, a first applicator (or electromagnetic coil) may be configured to stimulate a first region and/or first muscle and/or first muscle group and a second applicator (or electromagnetic coil) may be configured to stimulate a second region and/or second muscle and/or second muscle group. In particular, the first and the second applicator (or electromagnetic coil) may be triggered sequentially, or (substantially) simultaneously, e.g., based on (using) the above-described inventive circuit for generating a time-varying magnetic field. The skilled person will understand that the method may also be performed with more than two applicators and/or electromagnetic coils, e.g., four two applicators and/or electromagnetic coils. Likewise, the skilled person will understand that, irrespective of the number of applicators and/or electromagnetic coils used, the method may be performed based on (e.g., using) the above-described inventive circuit.

Hence, another aspect of the present invention is directed to an apparatus for inflammation reduction via magnetic stimulation. The apparatus comprises at least one applicator configured to generate a time-varying magnetic field to stimulate at least one muscle. The magnetic field (to be generated by the applicator) is adapted to trigger at least one contraction of the at least one muscle. The magnetic field (to be generated by the applicator) is further adapted to cover an area of at least 800 cm², preferably of at least 1.600 cm², most preferably of at least 3.000 cm² of a patient when the applicator is applied to the patient. Alternatively, or in addition, the magnetic field may be adapted to penetrate at least 5 cm, preferably at least 10 cm, most preferably at least 20 cm into at least one muscle of the patient when the applicator is applied to the patient.

In general, the applicator may be a hand-held device, comprising an electromagnetic coil configured, at least in part, to generate a time-varying magnetic field to stimulate at least one muscle. For example, the apparatus may comprise the above-described circuit. Part of the circuit, especially the electromagnetic coil, may be comprised in the applicator, while another part of the circuit may be comprised in another, spatially separate, entity, e.g., a main unit. In some embodiments, the apparatus may comprise the multichannel embodiment of the circuit. Furthermore, the apparatus may comprise multiple circuits. In addition, or alternatively, the apparatus may comprise multiple applicators. For example, a first applicator may be used to treat first muscles and/or muscle groups and a second applicator may be used to treat second muscles and/or muscle groups. In particular, the first muscles and/or muscle groups may be located at a different position than the second muscles and/or muscle groups. Specifically, the first muscles and/or muscle groups may be associated with the left glutes and the second muscles and/or muscle groups may be associated with the right glutes. In general, the applicator may form at least a part of a chair.

In general, the magnetic field may be adapted such as to trigger at least one contraction of at least one large muscle or muscle group of the patient exceeding 300 cm³, wherein preferably the at least one large muscle includes a gluteal muscle and/or a quadriceps of the patient.

Specifically, the apparatus may be adapted to generate the time-varying magnetic field such as to trigger and/or enhance myokine production in the patient.

A further aspect of the present invention relates to a use of an apparatus for magnetic stimulation for reducing inflammation, in particular low- and/or medium-grade chronic inflammation, in a patient.

In particular, the apparatus may be an apparatus as just described.

Apart from the stimulation of muscles performed in muscle stimulation magnetic therapies, magnetic stimulation may also be used for the stimulation of nerves and/or nerve centres. Therefore, another aspect relates to a method of magnetic stimulation, preferably for treating erectile dysfunction and/or premature ejaculation in a patient. The method comprises stimulating nerves and/or nerve centers associated with at least one of the vertebrae Th12, L1, L2, L3, S2, S3 and/or S4 of a spinal column of the patient. Generally, the method of magnetic stimulation may further comprise treating male infertility.

The nerves and/or the nerve centers of the patient may be stimulated by a time-varying magnetic field. For example, the time-varying magnetic field may be adapted to stimulate nerve and/or nerve centers associated with at least one of the vertebrae Th12, L1, L2, L3, S2, S3 and/or S4 of a spinal column of the patient. Specifically, the method may comprise to stimulate an area located about 15-20 cm from a lowest part of a spinal column of the patient and/or an area located about 28-30 cm from a lowest part of a spinal column of the patient and/or an area located about 36-40 cm from a lowest part of a spinal column of the patient

In general, stimulating nerves and/or nerve centers may be performed at least partially together with a stimulation of muscles. For example, the method may further comprise applying a time-varying magnetic field to at least one muscle of the patient to magnetically stimulate the at least one muscle, wherein the magnetic field is adapted such as to trigger at least one contraction of the at least one muscle. The time-varying magnetic field for stimulating the at least one muscle and/or muscle group may at least in part coincide with the time-varying magnetic field adapted to stimulate nerves and/or nerve centres. Specifically, the muscle and/or muscle groups may comprise at least a part of the pelvic floor muscles, for example, ischiocavernosus and/or bulbospongiosus.

Stimulating the nerves and/or nerve centres may comprise stimulating the nerves and/or nerve centres by a time-varying magnetic field at least in part generated by applicators. For example, the method may be performed by using two applicators.

In particular, a first applicator may be directed to stimulate nerve and/or nerve centres, for example at least one of the vertebrae Th12, L1, L2, L3, S2, S3 and/or S4 of a spinal column of the patient. The first applicator may be arranged such that it is located at a back of the patient. Further, a second applicator may be directed to stimulate muscle and/or muscle groups, for example, at least a part of the pelvic floor muscles. The second applicator may be arranged such that the second applicator is positioned below the pelvic floor muscles of the patient.

The first applicator and/or the magnetic field generated by the first applicator may be configured such as to stimulate at least a part of the vertebrae Th12, L1, L2, L3, S2, S3 and/or S4 of a spinal column of the patient. For example, the first applicator may be configured to stimulate a sacral region S2-S4 and/or a lumbar region L2-L3 and/or a region associated with the erectile reflex center Th12-L2. In general, the first applicator may be configured such as to stimulate at least a part of an area located at least 5 cm, preferably at least 10 cm, most preferably at least 15 cm and/or at most 60 cm, preferably at most 50 cm, most preferably at most 40 cm from the lowest part of the spinal column. In particular, the first applicator may be centered at about 25-30 cm from the lowest part of the spinal column of the patient.

Specifically, the first applicator may be adapted to stimulate an area of at least 8 cm width. In general, the first applicator may be circularly shaped. For example, the first applicator may comprise a circular shape. For example, the first applicator may have a diameter of at least 15 cm, preferably at least 20 cm, most preferably at least 25 cm. In addition, or alternatively, the first applicator may comprise an extended and/or elliptical shape. For example, the extended and/or elliptical shape may have an effective diameter of at least 15 cm, preferably at least 20 cm, most preferably at least 25 cm. The effective diameter may be associated with a semiaxis of the elongated and/or elliptical shape.

In some embodiments, the first applicator may comprise a plurality of applicators. For example, the plurality of applicators of the first applicator may be associated with a plurality of areas of the patient. In particular, the first applicator may comprise three applicators located in an area associated with the back of the patient. Each of the tree applicators may be configured to stimulate nerves and/or nerve centres. For example, there may be a bijective correspondence between the three applicators and the sacral region S2-S4, the lumbar region L2-L3 and the region associated with the erectile reflex center Th12-L2.

Alternatively, the first applicator may comprise two applicators. For example, one of the two applicators may be configured to stimulate nerve and/or nerve centres associated with S2-S4, and the other applicator may be configured to stimulate nerve and/or nerve centres associated with T12-L3.

The second applicator may comprise a circular and/or extended shape. For example, a diameter of the second applicator may be at least 15 cm, preferably at least 20 cm, most preferably at least 25 cm. In addition, or alternatively, the diameter of the applicator may be at most 50 cm, preferably at most 40 cm, most preferably at most 35 cm.

In general, the stimulation may be based at least in part on a time-varying magnetic field generated by the above-described circuit. For example, the first and/or the second applicator may comprise one embodiment of the above-described circuit. In particular, the first and/or the second applicator may comprise the multichannel circuit. For example, the first and the second applicator may be a multichannel circuit. Further, the first applicator may comprise a multichannel circuit. In particular, if the first applicator comprises multiple applicators, the different applicators may be arranged in a multichannel configuration. In addition, or alternatively, other types of LC circuits may be used.

In some embodiments, the treatment may be only directed to muscle stimulation or nerve stimulation. Alternatively, or in addition, the treatment may be directed to muscle and nerve stimulation.

The stimulation of nerves and/or nerve centers associated with at least one of the vertebrae Th12, L1, L2, L3, S2, S3 and/or S4 of a spinal column of the patient may be particularly well performed when the patient is in a sitting and/or lying position. For example, when treating erectile dysfunction and/or premature ejaculation and/or male infertility in a patient the patient may sit in a chair.

In general, the chair may be configured for magnetic stimulation, preferably for treating erectile disfunction and/or premature ejaculation in a patient. For example, the chair may be adapted to receive at least a part of a patient, preferably to receive the patient in a sitting position.

In general, at least one applicator may be placed over a penis of the patient. In addition, or alternatively, at least one applicator may be placed under the penis of the patient. For example, one applicator may be placed above the penis and one applicator may be placed under the penis. In particular, the at least one applicator may be placed at a distance from the penis of at most 1 cm, preferably at most 0.5 cm, most preferably in contact with the penis. In addition, or alternatively, the at least one applicator may be placed at a distance from the penis of at most 10 cm, preferably at most 2 cm.

In another embodiment, at least one applicator may be of a cylindrical, or other elongated shape where the applicator has an elongated opening with coils positioned around that opening, such that the penis can be inserted fully or at least partially into the opening.

Another aspect relates to a foot pedal and/or a handheld controlled switch configured for at least partially controlling a time-varying magnetic field for magnetic stimulation. For example, the foot pedal and/or the handheld controlled switch may be configured for controlling at least partially a magnetic stimulation for reducing inflammation and/or treating erectile dysfunction and/or premature ejaculation and/or male infertility. In particular, the foot pedal and/or the handheld controlled switch may be configured to control at least partially a magnetic stimulation of the method for reducing inflammation or in of the method for treating erectile dysfunction and/or premature ejaculation.

Controlling at least a part of a time-varying magnetic field for magnetic stimulation may comprise controlling a start of a magnetic field pulse and/or a start of a train of magnetic field pulses. In addition, or alternatively, the control may comprise a continuously triggering of magnetic field pulses. For example, magnetic field pulses may be triggered continuously, when the foot pedal and/or the handheld controlled switch is in an active state. In some embodiments, controlling at least a part of a time-varying magnetic field for magnetic stimulation may further comprise controlling an intensity of the time-varying magnetic field and/or other parameters associated with the magnetic stimulation.

Generally, the foot pedal and/or the handheld controlled switch may comprise at least one channel for adjusting at least one property. For example, a first property and/or a first set of properties may be adjusted by a first channel. In addition, a second property and/or a second set of properties may be adjusted by a second channel. The different channels may be independently adjustable. For example, the first channel may adjust the first property and/or the first set of properties independently of the second channel.

The foot pedal and/or the handheld controlled device may be configured such as to at least partially control the time-varying magnetic field for magnetic stimulation discretely. Controlling at least a part of the time-varying magnetic field discretely may comprise that the configuration of the magnetic field may be switched among a finite set of configurations. For example, the discrete control may comprise an on/off control of the magnetic field. In some embodiments, the discrete control may comprise a discrete control of the intensity and/or a length of a train of pulses. For example, the discrete control may allow to select among a set of predefined intensities and/or predefined lengths of the pulse train. In addition, or alternatively, the foot pedal and/or the handheld controlled device may be configured such as to at least partially control the time-varying magnetic field for magnetic stimulation continuously. Controlling at least a part of the time-varying magnetic field continuously may comprise that that the configuration of the magnetic field may be switched among a continuous set of configurations. A continuous set of configurations may be a set of configurations that comprises at least 50 configurations, preferably at least 100 configurations, more preferably at least 200 configurations, most preferably at least 1000 configurations. In general, a continuous set of configurations may be associated with a continuous adjustment of the footswitch and/or the handheld controlled device. For example, the foot switch may be a pressure-based foot switch such that the pressure is associated at least in part with a configuration of the time-varying magnetic field. In particular, continuously adjusting the foot switch, e.g., a pressure-based foot switch, may continuously control the intensity of the varying magnetic field.

In general, the foot pedal and/or the handheld controlled switch may be connected to a device for magnetic stimulation. The connection may comprise a wireless and/or a wired connection. The device may comprise at least one applicator. For example, the foot pedal and/or the handheld controlled switch may control one or more applicators. In particular, the foot pedal and/or the handheld controlled switch may control all applicators of the device. Alternatively, foot pedal and/or the handheld controlled switch may only control a subset of applicators of the device. The device may at least partially comprise the above-described inventive circuit.

In some embodiments, a multiple foot pedals and/or handheld controlled switches may be used. Each foot pedal and/or each handheld controlled device may independently control at least one parameter of the time-varying magnetic field. In addition, or alternatively, a combination of at least one foot pedal and at least one handheld controlled switch may be used. For example, one foot switch and one handheld controlled device may be used. In some embodiments, a first foot pedal and/or handheld controlled switch may control a first set of applicators of the device and a second foot pedal and/or handheld controlled switch may control a second set of applicators of the device.

Generally, the foot pedal and/or the handheld controlled switch makes treatment protocols more adaptable to the needs of both, the therapist and/or the patient. Using the foot pedal and/or the handheld controlled switch may synchronise the magnetic pulses with patient's movement and/or heartbeat and/or breathing. In addition, or alternatively, using a foot pedal and/or the handheld controlled switch may control the start/stop and intensity of the movement of the muscles as result of the magnetic stimulation. Additionally, the foot pedal and/or the handheld controlled switch may be used only to simplify the procedure for the therapist.

A magnetic stimulation device controlled with at least one foot pedal and/or handheld controlled device enables new modes of application of the technology. Whereas before all stimulation protocols were preset by therapist and during the therapy only the output intensity could be adjusted during the therapy via device, now the user can trigger pulses, sets the intensity, or modify other parameters at his own will or according to the therapy requirements without the need to move from the patient to the device and touching the device. This makes treatment protocols more adaptable to the needs of both, the user and the patient. Foot operated control also enables the user to freely manipulate applicator(s) during the therapy, thus making treatment options even more flexible.

Furthermore, another aspect relates to an apparatus for magnetic stimulation. The apparatus comprises a first element adapted to receive at least a part of a body of a patient and a first set of one or more electromagnetic coils arranged at the first element. The first element is adjustable such as to move relative to the first set of one or more electromagnetic coils. The apparatus may be adapted to receive and/or support the patient in a sitting or lying position.

Generally, the apparatus may be adapted to perform at least parts of the methods described herein. In particular, the apparatus may be adapted to perform at least partially the method for reducing inflammation in a patient as described herein. For example, at least a part of the coils of the first set of electromagnetic coils may be configured to apply a time-varying magnetic field to at least one muscle of the patient to magnetically stimulate the at least one muscle, wherein the magnetic field of the at least one electromagnetic coil of the first set of electromagnetic coils is adapted such as to trigger at least one contraction of the at least one muscle.

In addition, or alternatively, the apparatus may be adapted to perform at least partially the method for treating erectile dysfunction and/or premature ejaculation in a patient as described herein. For example, at least one electromagnetic coil of the firsts et of electromagnetic coils may be adapted to stimulate nerves and/or nerve centres associated with at least one of the vertebrae Th12, L1, L2, L3, S2, S3 and/or S4 of the spinal column of the patient. In addition, or alternatively, the apparatus may further be adapted to perform the method of magnetic stimulation for treating male infertility.

In addition, or alternatively, the apparatus may be adapted to perform the method for muscle contraction stimulation therapy as described herein, particularly for treating urinary incontinence.

The first element is configured to receive at least a part of a body of a patient. In particular, the first element may be adapted to receive and/or support the patient in a sitting or lying position. For example, the first element may be adapted to receive and/or support at least a part of a buttock and/or a back of the patient. In addition, or alternatively, the first element may be adapted to receive and/or support at least a part of a leg of the patient. Furthermore, a first set of electromagnetic coils may be arranged at the first element. Arranging a first set of electromagnetic coils at the first element may comprise to arrange the first set of coils in an area associated with the first element. For example, the first set of electromagnetic coils may be arranged in a vicinity of the first element. Arranging the first set of electromagnetic coils at the first element may not necessarily comprise that the first set of electromagnetic coils is in contact and/or mounted on the first element. The first element may be adjustable, in particular spatially adjustable, such as to move relative to the first set of one or more electromagnetic coils. Being adjustable may comprise that the first element is movable with respect to at least one direction relative to the first set of one or more electromagnetic coils. For example, the first element may be movable with respect to one direction relative to the first set of one or more electromagnetic coils.

Comprising a first element adapted to receive at least a part of a body of the patient such as to support the patient in a lying and/or sitting position may stabilize the position of the patient during magnetic stimulation, thereby relieving the patient and maximizing the comfort during the stimulation of the patient. In addition, stabilizing the position of the patient minimizes undesired displacements of the patient and particularly of the treatment area associated with the patient during the stimulation, thereby contributing to an optimal stimulation and/or treatment of the patient.

That the first element is adjustable such as to move relative to the first set of one or more electromagnetic coils may allow to move a position of at least a part of the body of the patient relative to the first set of one or more electromagnetic coils. In particular, when the first element is adapted to receive at least a part of the body of the patient such as to support the patient in a lying and/or sitting position, the adjustable first element may allow to move a position of at least a part of the body of the patient relative to the first set of one or more electromagnetic coils. Therefore, the position of at least a part of the patient relative to the first set of electromagnetic coils can be adjusted and/or controlled, thereby allowing to position at least a part of the body of the patient at an optimal position for stimulation and/or treatment. For example, the optimal position may be a position relative to the first set of one or more electromagnetic coils. In particular, the optimal position may be a position optimal for an individual patient, i.e., the optimal position may be determined by the individual patient, e.g., the size and/or proportions of the individual patient. For example, the optimal position may comprise an optimal distance to the first set of one or more electromagnetic coils. Consequently, the stimulation and/or treatment may be optimized.

In general, the apparatus may further comprise a frame, wherein the first element is movably attached to the frame and the first set of one or more magnetic coils is non-movably attached to the frame.

The first element may be movably attached to the frame. For example, being movable attached to the frame may comprise that the first element can be moved with respect to the frame in at least one direction. In particular, the first element may be movable with respect to the frame in one direction, preferably two directions, most preferably three directions. For example, the first element may be moveably attached to the frame such that the first element can be moved within a plane. Being movably attached to the frame may be at least in part based on guide rails. For example, the frame and/or the first element may comprise guide rails such as to movably attach the first element to the frame. The first set of one or more electromagnetic coils may be non-movably attached to the frame. In particular, the first set of one or more electromagnetic coils may be attached such to the frame that the first element is adjustable such as to move relative to the first set of one or more electromagnetic coils. For example, at least a part of the first set of one or more electromagnetic coils may be mounted on the frame.

By movably attaching the first element to the frame and by attaching the first set of one or more electromagnetic coils non-movably to the frame, the first element may be movable relative and independently to the first set of one or more electromagnetic coils. Thereby, the position of at least a part of the body of the patient may be moved relative to the first set of one or more electromagnetic coils.

The apparatus may form at least a part of a chair. The chair may further comprise a second element adapted to receive at least a part of the body of the patient, wherein the first element forms at least a part of a seating surface of the chair and the second element forms at least a part of a backrest of the chair. Alternatively, the first element may form at least a part of the backrest of the chair and the second element may form at least a part of the seating surface of the chair.

For example, the frame may be configured such as to form at least a part of the chair. In particular, the frame may be configured such as to form a base and/or frame of the chair. The first element may be configured to form at least a part of a seating surface of the chair. For example, the first element may be configured to receive at least a part of a buttock of the patient. In general, moving the position of the first element relative to the frame and/or the first set of electromagnetic coils may move a position of at least a part of the buttock of the patient relative to the frame and/or the first set of one or more electromagnetic coils.

By configuring the first element such as to form at least a part of a seating surface of the chair, the first set of one or more electromagnetic coils arranged at the first element may be located in a vicinity of the buttock of the patient. Therefore, the first set of one or more electromagnetic coils may stimulate and/or treat at least a part of the patient which is associated with the buttock. In particular, the first set of electromagnetic coils may be configured to stimulate at least a part of the buttock muscles of the patient, preferably at least a part of the pelvic floor muscles. In addition, or alternatively, at least one electromagnetic coil of the first set of electromagnetic coils may be placed below a penis of the patient, e.g., by adjusting the position of at least a part of the seating surface.

The apparatus, particularly the chair, may comprise a second element adapted to receive at least a part of the body of the patient. The second element may form at least a part of a backrest of the chair. For example, the second element may be configured to receive at least a part of a back of the patient. In general, the second element may be mounted on the frame. For example, the second element may be movable mounted to the frame. In particular, the second element may be movable relative to the frame and/or to the first element. For example, the second element may be pivotable relative to the frame and/or the first element. In some embodiments, the second element is not movable relative to the first element and/or the frame. For example, the second element may be comprised in the frame.

By movably mounting the second element to the chair, in particular pivotable relative to the first element, the position of the back of the patient and/or an inclination of the back of the patient relative to the first element may be adjusted. This may allow the patient to sit in a pleasant position in the chair and thereby maximizing the comfort of the patient during stimulation and/or treatment. Further, adjusting the inclination of the back of the patient relative to the first element may allow to change an inclination of at least a part of the patient relative to the first set of one or more electromagnetic coils, thereby allowing to move at least a part of the patient into a position optimal for stimulation and/or treatment.

Alternatively, the first element may form the backrest of the chair and the second element may form at least a part of the seating surface of the chair. Thereby, the first set of one or more electromagnetic coils arranged at the first element may be located in an area associated with the back of the patient. In particular, the first set of one or more electromagnetic coils may be located in a vicinity of the back of the patient. In this case, being movable relative to the first set of one or more electromagnetic coils may comprise to change a position of the first element relative to the first set of one or more electromagnetic coils. For example, moving the first element relative to the first set of one or more electromagnetic coils may comprise to move at least a part of the back of the patient relative to the first set of one or more electromagnetic coils.

Specifically, the apparatus may further comprise a second set of one or more magnetic coils arranged at the second element. In particular, the second element may be adjustable such as to move relative to the second set of one or more magnetic coils. Alternatively, or in addition, at least one magnetic coil of the second set may be adjustable such as to move relative to the second element.

The second set of one or more electromagnetic coils may be arranged at the second element. Being arranged at the second element may comprise that the second set of one or more electromagnetic coils is located in a vicinity of the second element. In particular, when the second element is adapted to form at least a part of the backrest of the chair, the second set of one or more electromagnetic coils may be arranged such as being located in a vicinity of the back of the patient. Specifically, being located in a vicinity of the back of the patient may comprise an area such that the second set of one or more coils can magnetically stimulate at least a part of the back of the patient. Similarly, when the second element is adapted to form at least a part of the seating surface of the chair, the second set of one or more electromagnetic coils may be arranged such as being located in a vicinity of the buttock of the patient. Specifically, being located in a vicinity of the buttock of the patient may comprise an area such that the second set of one or more coils can magnetically stimulate at least a part of the buttock of the patient. In general, at least a part of the second set of one or more electromagnetic coils may be comprised in the second element. In addition, or alternatively, at least a part of the second set of one or more electromagnetic coils may be located at a rear panel of the second element. A rear panel of the second element may a side of the second element which is opposite to a side of the second element adapted to receive at least the part of the body of the patient.

The second element may be adjustable such as to move relative to the second set of electromagnetic coils. By moving the second element relative to the second set of one or more electromagnetic coils a position of at least a part of the body of the patient may be moved relative to the second set of one or more electromagnetic coils. Thereby, different parts of the body of the patient may be magnetically stimulated and/or treated. In particular, moving at least a part of the body of the patient relative to the second set of one or more electromagnetic coils, the at least one part of the body of the patient may be moved in an optimal treatment position. For example, the at least part of the second set of one or more electromagnetic coils may be non-movably attached to the frame and the second element may be adjusted by moving the second element relative to the frame and/or the second set of one or more electromagnetic coils.

Alternatively, or in addition, at least one magnetic coil of the second set may be adjustable such as to move relative to the second element. For example, at least one electromagnetic coil of the second set may be movable independent from the second element. In particular, the at least one coil may be movable relative to the second element with respect to at least one direction. In some embodiments, the at least one coil may be movable with respect to two directions, preferably three directions relative to the second element. Generally, the second element may be movable relative to the frame and/or the first element and at least one electromagnetic coil of the second set is adjustable such as to move relative to the second element. In some embodiments, the second element may be non-movable, when at least one electromagnetic coil of the second set is adjustable such as to move relative to the second element.

Generally, the one or more electromagnetic coils in the first and/or in the second set may be at least in part comprised in a circuit as described above. For example, at least one electromagnetic coil of the first set may be comprised in an above-described LC circuit. For example, at least one electromagnetic coil may be comprised in the multichannel circuit. In particular, the one or more electromagnetic coils in the first set may be comprised in a first multichannel circuit. In addition, or alternatively, the one or more electromagnetic coils in the second set may be comprised in a second multichannel circuit. In some embodiments, the one or more electromagnetic coils in the first set and the second set may be comprised in a multichannel circuit.

Generally, the apparatus, in particular at least one magnetic coil, may be adapted to stimulate a lower part of the back of the patient.

Stimulating a part of the patient may comprise to submit at least a lower part of the patient to a time-varying magnetic field. In particular, the time-varying magnetic field may be generated by the at least one electromagnetic coil. For example, stimulating a lower part of the back of the patient may comprise to stimulate muscles and/or muscle groups and/or nerves and/or nerve centers associated with the lower part of the back of the patient.

Specifically, the apparatus, in particular at least one magnetic coil, may be adapted to stimulate nerves and/or nerve centers associated with at least one of the vertebrae Th12, L1, L2, L3, S2, S3 and/or S4.

Stimulating nerves and/or nerve centers associated with at least one of the vertebrae Th12, L1, L2, L3, S2, S3 and/or S4 may comprise to submit at least one of the vertebrae to the time-varying magnetic field of the at least one electromagnetic coil. Stimulating nerves and/or nerve centers associated with at least one of the vertebrae Th12, L1, L2, L3, S2, S3 and/or S4 may stimulate the parasympathetic and/or the sympathetic reflex center and/or the pelvic floor muscles. The stimulation may increase the blood flow in a region associated with the stimulation and/or may increase the elastin deposits in the cavernous bodies and/or may increase venous occlusion by strengthening the pelvic floor muscles.

The first set and/or the second set may comprise two or more electromagnetic coils. For example, the first set may comprise at least two electromagnetic coils arranged at the first element. In particular, the at least two electromagnetic coils may be arranged in a region associated with the buttock of the patient, preferably when the first element forms at least a part of the seating surface of the chair. Similarly, the second set may comprise at least two electromagnetic coils. For example, the second set may comprise at least two electromagnetic coils arranged at the second element. In particular, the at least two electromagnetic coils may be arranged in a region associated with the back of the patient, preferably when the second element forms at least a part of the backrest of the chair. In some embodiments, only the first set may comprise two or more electromagnetic coils. Alternatively, only the second set may comprise two or more electromagnetic coils.

Generally, the two or more electromagnetic coils may be adapted to stimulate two or more areas associated with the body of the patient. For example, a first electromagnetic coil may be adapted to stimulate a first region and a second electromagnetic coil may be adapted to stimulate a second region. In particular, the first and the second region may be connected. Alternatively, the first and the second region may be unconnected and/or separated by a non-stimulated area.

Using a first set and/or the second set that comprise two or more electromagnetic coils, may allow to stimulate and/or to treat larger areas of the body of the patient. For example, using two or more electromagnetic coils in the second set may allow to treat at least a large part of the vertebrae Th12, L1, L2, L3, S2, S3 and/or S4. For example, a large part of the vertebrae may comprise at least two, preferably at least three, more preferably at least four, most preferably all the vertebrae Th12, L1, L2, L3, S2, S3 and/or S4. In addition, or alternatively, using two or more electromagnetic coils may allow for stimulating disjoint areas of the body of the patient. Generally, the two or more electromagnetic coils may be independent of each other. For example, a first electromagnetic coil may generate a time-varying electromagnetic field independently from a second electromagnetic coil. In particular, the first electromagnetic coil may generate a time-varying magnetic field while the second electromagnetic coil does not generate a time-varying magnetic field.

A first magnetic coil of the apparatus may be adapted to stimulate nerves and/or nerve centers associated with at least one of the vertebrae Th12, L1, L2 and/or L3 of a spinal column of the patient. In addition, a second magnetic coil may be adapted to stimulate nerves and/or nerve centers associated with at least one of the vertebrae S2, S3 and/or S4 of the spinal column of the patient.

For example, the first electromagnetic coil may be arranged at the first element. In particular, the first electromagnetic coil may be arranged such at the first element that the generated time-varying electromagnetic field may stimulate nerves and/or nerve centers associated with at least one of the vertebrae Th12, L1, L2 and/or L3 of a spinal column of the patient. Similarly, the second electromagnetic coil may be arranged at the first element. In particular, the second electromagnetic coil may be arranged such at the first element that the generated time-varying electromagnetic field may stimulate nerves and/or nerve centers associated with at least one of the vertebrae S2, S3 and/or S4 of the spinal column of the patient. The first electromagnetic coil be arranged such at the first element as to be positioned above the second electromagnetic coil. For example, the first electromagnetic coil may be arranged above the second electromagnetic coil such that the first and the second electromagnetic coil are not shifted relative to each other.

Specifically, the first magnetic coil may be adapted to stimulate at least a part of an area located at least 10 cm, preferably at least 20 cm, most preferably at least 28 cm and/or at most 60 cm, preferably at most 50 cm, most preferably at most 40 cm from the lowest part of the spinal column. In addition, or alternatively, the second magnetic coil may be adapted to stimulate at least a part of an area located at least 5 cm, preferably at least 10 cm, most preferably at least 15 cm and/or at most 40 cm, more preferably at most 30 cm, most preferably at most 20 cm from the lowest part from the spinal column.

For example, the first electromagnetic coil may be arranged such at the first element as to stimulate a part of an area located at least at least 10 cm, preferably at least 20 cm, most preferably at least 28 cm from the lowest part of the spinal column of the patient. In addition, or alternatively, the first electromagnetic coil may be arranged such at the first element as to stimulate a part of an area located at most 60 cm, preferably at most 50 cm, most preferably at most 40 cm from the lowest part of the spinal column. Such an arrangement of the first electromagnetic coil may allow to stimulate nerves and/or nerve centers associated with at least one of the vertebrae Th12, L1, L2 and/or L3 of a spinal column of the patient. In particular, such an arrangement may allow to stimulate and/or treat at least a part of the sympathetic erectile reflex center and/or centers for ejaculation and/or orgasm.

Similarly, the second electromagnetic coil may be arranged such at the first element as to stimulate a part of an area located at least 5 cm, preferably at least 10 cm, most preferably at least 15 cm from the lowest part from the spinal column of the patient. In addition, or alternatively, second electromagnetic coil may be arranged such at the first element as to stimulate a part of an area located at most 40 cm, more preferably at most 30 cm, most preferably at most 20 cm from the lowest part from the spinal column of the patient. Such an arrangement of the second electromagnetic coil may allow to stimulate nerves and/or nerve centers associated with at least one of the vertebrae S2, S3 and/or S4 of the spinal column of the patient. In particular, such an arrangement may allow to stimulate and/or treat at least a part of the parasympathetic reflex center and/or the sacral region of the patient. In addition, or alternatively, the arrangement may also allow for stimulating at least a part of the pelvic floor muscles.

In particular, the first coil may be adapted to stimulate an area of at least 8 cm width. In addition, or alternatively, the second coil may be adapted to stimulate an area of at least 6 cm width.

Stimulating with the first coil an area of at least 8 cm width may ensure to stimulate at least a part of the nerves and/or nerve centers associated with at least one of the vertebrae Th12, L1, L2 and/or L3 of a spinal column of the patient. Further, an area of at least 8 cm width may ensure that at least a part of the nerves and/or nerve centers associated with at least one of the vertebrae Th12, L1, L2 and/or L3 of a spinal column of the patient is stimulated. For example, an area comprising at least 60 %, preferably at least 70 %, more preferably at least 80 %, most preferably at least 90 % of the nerves and/or nerve centers associated with at least one of the vertebrae Th12, L1, L2 and/or L3 of a spinal column of the patient may be stimulated. Therefore, the stimulation of at least one of the vertebrae Th12, L1, L2 and/or L3 of a spinal column may be maximized and thereby optimizing the efficiency of the treatment. Similarly, an area of at least 6 cm width may ensure that at least a part of the nerves and/or nerve centers associated with at least one of the vertebrae S2, S3 and/or S4 of the spinal column of the patient is stimulated.

Generally, a first magnetic coil may be adapted to stimulate nerves and/or nerve centers associated with at least one of the vertebrae Th12, L1, L2 and/or L3 as well as nerves and/or nerve centers associated with at least one of the vertebrae S2, S3 and/or S4 of a spinal column of the patient. Specifically, the first magnetic coil may be adapted to stimulate at least a part of an area located at least 5 cm, preferably at least 10 cm, most preferably at least 15 cm and/or at most 60 cm, preferably at most 50 cm, most preferably at most 40 cm from the lowest part of the spinal column. In addition, or alternatively, the first magnetic coil may be adapted to stimulate an area of at least 10 cm, preferably at least 15 cm, most preferably at least 20 cm and/or at most 40 cm, preferably 30 cm, most preferably at most 22 cm width.

Adapting the first magnetic coil to stimulate at least a part of an area located at least 5 cm, preferably at least 10 cm, most preferably at least 15 cm may ensure that at least a part of the vertebrae Th12, L1, L2 and/or L3 and/or S2, S3 and/or S3 of a spinal column of the patient is stimulated. Similarly, adapting the first magnetic coil to stimulate at least a part of an area located at most 60 cm, preferably at most 50 cm, most preferably at most 40 cm from the lowest part of the spinal column may ensure that at least a part of the vertebrae Th12, L1, L2 and/or L3 and/or S2, S3 and/or S3 of a spinal column of the patient is stimulated. For example, the first element may only comprise a first electromagnetic coil. In particular, the first electromagnetic coil may be adapted to stimulate at least a part of the vertebrae Th12, L1, L2, L3 and at least a part of the vertebrae S2, S3, S3 of the spinal column of the patient.

In particular, the first magnetic coil may comprise an elongated, preferably elliptical, shape. In particular, the first electromagnetic coil may be elongated, preferably elliptical, with respect to an elongation of the spinal column of the patient. For example, a first semiaxis essentially parallel to a direction of the spinal column of the patient when sitting and/or lying in the chair may be larger than a second semiaxis of the first electromagnetic coil. The first semiaxis of the first electromagnetic coil may comprise an effective diameter of at least 15 cm, preferably at least 20 cm, most preferably at least 25 cm.

In general, the one or more electromagnetic coils in the second set may be at least in part adapted to stimulate muscles. In particular, the configuration of the one or more electromagnetic coils in the second set may be essentially the same as the configuration of the at least one electromagnetic coil adapted for the treatment of inflammation. For example, the range of the magnetic field amplitudes may be essentially the same as for the at least one electromagnetic coil adapted for the treatment of inflammation.

Similarly, the repetition frequencies may be essentially the same as for the at least one electromagnetic coil adapted for the treatment of inflammation. In particular, the repetition frequency may be at least 3 Hz, preferably at least 5 Hz, most preferably at least 40 Hz. In addition, or alternatively, the repetition frequency may be at most 100 Hz, preferably at most 50 Hz, most preferably at most 40 Hz.

Furthermore, at least one of the electromagnetic coils in the first set may be adapted for stimulating muscles and/or nerves and/or nerve centres. In general, the repetition frequency of at least one electromagnetic coil may be associated with a normal operating mode. In the normal operating mode, the repetition frequency of the at least one electromagnetic coil in the first set may be at least 3 Hz, preferably at least 5 Hz, most preferably at least 10 Hz. In addition, or alternatively, the repetition frequency of at least one electromagnetic coil of the first set may be at most 100 Hz, preferably at most 50 Hz, most preferably at most 40 Hz. In some embodiments, particularly in embodiments directed to intense nerve stimulation, the repetition frequency of at least one electromagnetic coil may be associated with an intense operating mode. In the intense operating mode, the repetition frequency of the at least one electromagnetic coil may be at least 20 Hz, preferably at least 30 Hz, most preferably at least 40 Hz. In addition, or alternatively, the repetition frequency may be at most 400 Hz, preferably at most 300 Hz, most preferably at most 200 Hz. In general, a first electromagnetic coil of the first set may be configured to operate according to the normal operating mode and a second electromagnetic coil of the first set may be configured to operate according to the intense operating mode.

In addition, or alternatively, the apparatus, particularly the chair, may comprise a third set of electromagnetic coils. In general, the third set of electromagnetic coils may comprise at least one electromagnetic coil. For example, the third set of electromagnetic coils may be arranged at the seating surface of the chair. In particular, the third set of electromagnetic coils may be arranged below the seating surface and/or below a cushioning, e.g., at a side of the seating surface and/or the cushioning opposed to a side configured to receive a buttock of the patient. In some embodiments, at least a part of the third set of electromagnetic coils may be arranged within the cushioning of the seating surface. Generally, at least one coil of the third set of electromagnetic coils may be mounted to the frame of the chair. For example, the at least one electromagnetic coil of the third set of electromagnetic coils may be mounted to the chair, e.g., the frame of the chair, such as to enable at least a part of the seating surface to move relative to the at least one electromagnetic coil. In particular, the at least one electromagnetic coil may be mounted in such a way to the chair as to enable the part of the seating surface to move horizontally with respect to the at least one electromagnetic coil.

Generally, the at least one electromagnetic coil of the third set of electromagnetic coils may at least partially comprise the above-described circuit for generating a time-varying magnetic field. For example, the at least one electromagnetic coil of the third set of electromagnetic coils may comprise the at least a part of the multi-channel embodiment of the above-described circuit. For example, the third set of electromagnetic coils may comprise at least two electromagnetic coils, wherein a first electromagnetic coil corresponds to a first channel and a second electromagnetic coil corresponds to a second channel of the multichannel circuit.

In general, the third set of electromagnetic coils may be configured for treating inflammation and/or for body muscle strengthening. Being configured for treating inflammation and/or for body muscle strengthening may comprise that at least one electromagnetic coil of the third set of electromagnetic coils is adapted to treat and/or to stimulate an area of the patient associated with a large muscle and/or muscle group. For example, at least one electromagnetic coil of the third set of electromagnetic coils may be arranged and/or adapted such as to stimulate muscles and/or muscle groups associated with a thigh of the patient. Preferably, the muscles and/or muscle groups associated with the thigh may comprise at least one large muscle and/or muscle group of the thigh. For example, muscle and/or muscle groups associated with the thigh of the patient may comprise hamstrings and/or pectineus and/or quadriceps.

In general, the at least one electromagnetic coil of the first set and/or the second set and/or the third set of electromagnetic coils may be arranged at a distance to the patient of at most 15 cm, preferably at most 12 cm, more preferably at most 9 cm, even more preferably at most 6 cm, most preferably at most 3 cm. In particular, the at least one electromagnetic coil of the first set and/or the second set and/or the third set of electromagnetic coils may be essentially placed directly over the patient. For example, being placed essentially directly over the patient may comprise that the at least one electromagnetic coil is placed as close as possible to the patient. Specifically, the at least one electromagnetic coil may be placed at a distance to the patient of at most 2 cm, even more preferably at most 1 cm, most preferably at most 0.5 cm. Placing the at least one electromagnetic coil at a distance to the patient may comprise that the at least one electromagnetic coil is placed at a distance to a target and/or treatment region of the patient. In some embodiments, the at least one electromagnetic coil of the first set and/or second set and/or third set of electromagnetic coils may be at least partially comprised in a housing, e.g., a housing at least partially enclosing the at least one electromagnetic coil. Placing the at least one electromagnetic coil at said distances to the patient may comprise placing the housing at these distances to the patient, e.g., at the distance to the target and/or treatment region of the patient. Specifically, the at least one electromagnetic coil and/or the housing of the at least one electromagnetic coil may be arranged such as to contact the patient, e.g., the target and/or treatment region of the patient. Contacting the patient may comprise that the at least one electromagnetic coil and/or the housing of the at least one electromagnetic coil may directly contact a skin of the patient, e.g., a portion of the skin of the patient associated with a target and/or treatment region of the patient. In addition, or alternatively, contacting the patient may comprise that the at least one electromagnetic coil and/or the housing of the at least one electromagnetic coil contacts a clothing of the patient, e.g., the at least one electromagnetic coil and/or the housing of the at least one electromagnetic coil and/or the housing of the at least one electromagnetic coil and/or the housing of the at least one electromagnetic coil may essentially only be separated from the skin of the patient by the clothing of the patient covering the skin associated with the target and/or treatment region.

Arranging the at least one electromagnetic coil of the first and/or the second and/or the third set of electromagnetic coils at a distance to the patient of at most 15 cm, preferably at most 12 cm, more preferably at most 9 cm, even more preferably at most 6 cm, most preferably at most 3 cm may enhance the stimulation of muscles and/or muscle groups of the patient, thereby optimizing the success of the treatment. In addition, or alternatively, arranging the at least one electromagnetic coil of the first and/or the second and/or the third set of electromagnetic coils at a distance to the patient of at most 15 cm, preferably at most 12 cm, more preferably at most 9 cm, even more preferably at most 6 cm, most preferably at most 3 cm may enhance the stimulation of nerves and/or nerve centres of the patient, thereby optimizing the success of the treatment. In particular, placing the at least one electromagnetic coil sufficiently close to the patient may ensure that the electromagnetic field generated by the at least one electromagnetic coil is sufficiently strong at the position of the patient, e.g., at the position of the target area and/or treatment region. For example, the field strength of the electromagnetic field generated by the at least one electromagnetic coil at the position of the patient may attenuate with the distance between the at least one electromagnetic coil and the position of the patient, e.g., the target and/or treatment region. Therefore, placing the at least one electromagnetic coil sufficiently close to the patient, e.g., the target and/or the treatment region, may enable and/or optimize the treatment of muscles and/or muscle groups and/or nerves and/or nerve centres deeply placed within the body of the patient. For example, placing the at least one electromagnetic coil sufficiently close to the patient may enable and/or optimize the treatment of muscles and/or muscle groups and/or nerves and/or nerve centres placed at least 0.5 cm deep, preferably at least 0.75 cm deep, more preferably at least 1 cm deep, even more preferably at least 1.25 cm deep, most preferably at least 1.5 cm deep. In particular, placing the at least one electromagnetic coil sufficiently close to the patient may enable and/or optimize the treatment of overweight and/or obese patients, where the muscles and/or muscle groups and/or nerves and/or nerve centres are separated from the skin by adipose tissue. Specifically, adipose tissue may attenuate the field strength of the electromagnetic field generated by the at least one electromagnetic coil as the electromagnetic field propagates through the adipose tissue.

In addition, or alternatively, the distance of the at least one electromagnetic coil of the first and/or second and/or third set of electromagnetic coils to the patient may be at least partially based on a state of the patient. For example, if the patient requires intensive care, e.g., the patient suffers from severe illness and injuries, the at least one electromagnetic coil may be arranged at a distance to the patient of at least 0.5 mm, preferably at least 1 mm, more preferably at least 1.5 mm, most preferably at least 2 mm. For example, the at least one electromagnetic coil may be arranged at said distances when the patient is not able to report about his well-being and/or comfort during the treatment, and may thus contribute to an increased safety of the treatment.

Generally, the at least one electromagnetic coil of the third set of electromagnetic coils may be positioned such as to be located in a region of the seating surface associated with the thigh of the patient. For example, the at least one electromagnetic coil may be arranged at a right side of the chair and/or frame. Being arranged at a right side of the chair and/or frame may comprise that the at least one electromagnetic coil is located at a region associated with a right outer area, e.g., an area in a vicinity of a right outer end, of the seating surface.

In addition, or alternatively, at least one electromagnetic coil of the third set of electromagnetic coils may be arranged at a left side of the chair and/or frame. Being arranged at a left side of the chair and/or frame may comprise that the at least one electromagnetic coil is located at a region associated with a left outer area, e.g., an area in a vicinity of a left outer end, of the seating surface. For example, at least one electromagnetic coil of the third set of electromagnetic coils may be arranged at a left side of the chair and/or frame for stimulating muscles and/or muscle groups associated with a left thigh of the patient and at least one electromagnetic coil of the third set of electromagnetic coils may be arranged at a right side of the chair and/or frame for stimulating muscles and/or muscle groups associated with a right thigh of the patient.

Generally, at least one electromagnetic coil of the third set of electromagnetic coils may comprise a geometry and/or size adapted for stimulating muscles and/or muscle groups associated with a thigh of a patient. A geometry and/or a size adapted for stimulating muscles and/or muscle groups associated with a thigh of a patient may comprise that the geometry and/or the size of the at least one electromagnetic coil essentially corresponds to a geometry and/or a size of muscles and/or muscle groups associated with a thigh. For example, the size of the at least one electromagnetic coil may correspond to a length and/or a width of the thigh of the patient, preferably to a length of muscles and/or muscle groups of the thigh. For example, the at least one electromagnetic coil may comprise a length of at least 15 cm, preferably at least 18 cm, more preferably at least 21 cm, even more preferably at least 24 cm, most preferably at least 27 cm. In addition, or alternatively, the at least one electromagnetic coil may comprise a width of at least 5 cm, preferably at least 7 cm, more preferably at least 9 cm, most preferably at least 11 cm.

Generally, the geometry of the at least one electromagnetic coil may comprise an elliptical geometry or generally an elongated geometry along the direction of the thigh. For example, an elliptical electromagnetic coil may be arranged such that a semi-major axis of the ellipse is essentially aligned with an extension direction of the thigh of the patient, preferably when the patient sits on the seating surface. Specifically, the semi-major axis of the elliptical geometry may be adapted to the length of the thigh of the patient. For example, the semi-major axis may be at least 15 cm, preferably at least 18 cm, more preferably at least 21 cm, even more preferably at least 24 cm, most preferably at least 27 cm. In addition, or alternatively, the semi-minor axis of the elliptical geometry may correspond to a width of the thigh of the patient. For example, the semi-minor axis of the elliptical geometry may be at least 5 cm, preferably at least 7 cm, more preferably at least 9 cm, even more preferably at least 11 cm most preferably at least 13 cm. In addition, or alternatively, the semi-minor axis of the elliptical geometry may be at most 15 cm, preferably at most 13 cm, more preferably at most 11 cm, most preferably at most 9 cm.

In some embodiments, at least one electromagnetic coil of the third set of electromagnetic coils may be movable relative to at least one electromagnetic coil of the first set of electromagnetic coils. For example, the at least one electromagnetic coil of the third set may be movable relative to the at least one electromagnetic coil of the first set of electromagnetic coils such as to vary a distance, preferably a distance in a horizontal plane, between the at least one electromagnetic coil of the third set and the at least one electromagnetic coil of the first set. Specifically, the electromagnetic coils of the third set of electromagnetic coils may be jointly movable relative to first set of electromagnetic coils. In addition, or alternatively, at least one electromagnetic coil of the third set of electromagnetic coils may be movable relative to the third set of electromagnetic coils. For example, the electromagnetic coils of the third set may be movable such as to vary a distance between the electromagnetic coils of the third set of electromagnetic coils. Specifically, the electromagnetic coils of the third set may be movable such as to vary a distance between the electromagnetic coil associated with the left thigh of the patient and the electromagnetic coil associated with the right thigh of the patient. In particular, the electromagnetic coil associated with left thigh of the patient and the electromagnetic coil associated with the right thigh may be movable relative to a centre and/or midpoint of the chair and/or frame and/or seating surface. For example, the electromagnetic coil associated with left thigh of the patient and the electromagnetic coil associated with the right thigh may be jointly movable, e.g., varying a distance of the electromagnetic coil associated with the left thigh relative to the centre and/or midpoint may comprise varying a distance of the electromagnetic coil associated with the right thigh relative to the centre and/or midpoint.

In some embodiments, at least one electromagnetic coil of the first set of electromagnetic coils may be arranged at a rear end of the chair and/or seating surface and/or frame. In particular, the at least one electromagnetic coil of the first set of electromagnetic coils may be arranged essentially centrally at the rear end. Being arranged essentially centrally may comprise that the at least one electromagnetic coil of the first set of electromagnetic coils may be arranged at a mid-portion relative to the left and right side of the chair and/or frame and/or seating surface. In addition, or alternatively, at least one electromagnetic coil of the third set of electromagnetic coils may be arranged in front of the electromagnetic coil of the first set of electromagnetic coils. For example, the at least one electromagnetic coil of the third set of electromagnetic coils may be arranged in front of the electromagnetic coil of the first set of electromagnetic coils at a distance of at least 1 cm, preferably at least 5 cm, more preferably at least 10 cm, more preferably at least 15 cm, most preferably at least 20 cm. Specifically, the at least one electromagnetic coil of the third set may be arranged such in front of the at least one electromagnetic coil of the first set as form an angle with respect to the at least one electromagnetic coil of the first set of at least 5°, preferably at least 7°, more preferably at least 9°, even more preferably at least 11°, most preferably at least 13°. Generally, the third set of electromagnetic coils may comprise at least two electromagnetic coils, e.g., one electromagnetic coil arranged at a right side of the chair and one electromagnetic coil arranged at a left side of the chair. Specifically, the at least two electromagnetic coils of the third set of electromagnetic coils may be arranged essentially symmetric with respect to the at least one electromagnetic coil of the first set. Being arranged essentially symmetric may comprise that one electromagnetic coil of the third set of electromagnetic coils is arranged on the left side and one electromagnetic coil of the third set of electromagnetic coils is arranged on the right side such as to span essentially the same angle with respect to the at least one electromagnetic coil of the first set of electromagnetic coils.

Generally, the apparatus may further comprise at least one electromagnetic coil adapted to stimulate muscles and/or muscle groups associated with an abdomen of the patient. For example, stimulating muscles and/or muscle groups associated with the abdomen may comprise external obliques, internal obliques, rectus abdominis and/or transversus abdominis. Specifically, the at least one electromagnetic coil may be adapted to be arranged over the abdomen of the patient. For example, the at least one electromagnetic coil may be arranged at a distance over the abdomen of the patient of at least 1 cm, preferably at least 2 cm, more preferably at least 4 cm, even more preferably at least 5 cm, most preferably at least 6 cm.

In addition, or alternatively, the at least one electromagnetic coil may be arranged at a distance over the abdomen of the patient of at most 9 cm, preferably at most 7 cm, more preferably at most 5 cm, even more preferably at most 3 cm, most preferably at most 1 cm. In particular, the at least one electromagnetic coil may be essentially directly placed over the abdomen. For example, a housing comprising the at least one electromagnetic coil may be placed over the abdomen of the patient such as to contact the abdomen of the patient. Specifically, the at least one electromagnetic coil may be placed as close as possible over the abdomen of the patient.

Generally, the at least one electromagnetic coil may be comprised in an element adapted to be placed over the abdomen of the patient. For example, the element may comprise a curvature such as to essentially follow the shape of the abdomen of the patient. The element may be at least partially attached to the chair and/or frame. Specifically, the element may be mounted to the chair and/or the frame. In particular, the element may be pivotable mounted to the chair and/or the frame. For example, the element may be mounted to the chair and/or frame such as to enable a patient to sit on the chair when the element is in an open position. In addition, or alternatively, the element may be mounted to the chair and/or frame such as to be placed over the abdomen of the patient when the element is in a closed position. Generally, the element may be pivotable mounted to the chair and/or frame such that the element can pivot between the open and the closed position. In some embodiments, the element may be a separate element, e.g., not directly connected and/or attached to the chair.

### Short description of the figures

In the following, exemplary embodiments of the invention are described with reference to the following figures:
- **Fig. 1:**: Illustration of a prior art circuit for generating a time-varying magnetic field with a single channel and a resistor.
- **Fig. 2:**: Illustration of a prior art circuit for generating a time-varying magnetic field with a single channel without a resistor.
- **Fig. 3:**: Illustration of a prior art circuit for generating a time-varying magnetic field with multiple channels without a resistor.
- **Fig. 4:**: Exemplary embodiment of a single channel circuit for generating a time-varying magnetic field according to the present invention.
- **Fig. 4A:**: Exemplary embodiment of a capacitor bank according to the present invention.
- **Fig. 5A:**: Exemplary embodiment of a circuit configured for triggering a switch comprising an optocoupler according to the present invention.
- **Fig. 5B:**: Exemplary embodiment of a circuit configured for triggering a switch comprising a trigger transformer according to the present invention.
- **Fig. 6:**: Exemplary embodiment of a current waveform and corresponding trigger signals for a first switch according to the present invention.
- **Fig. 7:**: Exemplary embodiment of a multichannel circuit for generating a time-varying magnetic field according to the present invention.
- **Fig. 8:**: Exemplary embodiment of a multichannel circuit for generating a time-varying magnetic field according to the present invention.
- **Fig. 9:**: Exemplary embodiment of circuit adapted to reduce working voltages according to the present invention.
- **Fig. 10:**: Dependence of the current I (in mA) induced in a given muscle tissue on the distance d and the magnetic field Bₒ (in Tesla).
- **Fig. 11A**:: Right side view of a chair for magnetic stimulation, particularly for treating erectile dysfunction and/or premature ejaculation in a patient;
- **Fig. 11B:**: Left side view of the chair for magnetic stimulation, particularly for treating erectile dysfunction and/or premature ejaculation in a patient;
- **Fig. 11C:**: Left side top view of the chair for magnetic stimulation, particularly for treating erectile dysfunction and/or premature ejaculation in a patient;
- **Fig. 11D:**: Front top view of the chair for magnetic stimulation, particularly for treating erectile dysfunction and/or premature ejaculation in a patient;
- **Fig. 11E:**: Detailed left side view of the chair for magnetic stimulation, particularly for treating erectile dysfunction and/or premature ejaculation in a patient.

### Detailed description of preferred embodiments

Figure 4 illustrates an exemplary embodiment of a circuit 400 according to one aspect of the present invention. The circuit 400 comprises a capacitor bank 410, a first electromagnetic coil 420, a power supply 430, a switch 440, a resistance 450 and a first switch 460. The electromagnetic coil 420 and the capacitor bank 410 are arranged such as to form a first LC circuit. The power supply 430 is configured to charge the capacitor bank 410 by applying a charging voltage. The polarity of the charging voltage is indicated by +/- signs at the power supply 430. In other embodiments, the charging voltage may be of reverse polarity as compared to what is shown in Figure 4. The switch 440 is configured to electrically disconnect the first LC circuit comprising the first electromagnetic coil 420 and the capacitor bank 410 from the power supply 430 when a voltage across the capacitor bank 410 is reverse in polarity to the charging voltage of the power supply 430.

The capacitor bank 410 (as well as the first electromagnetic coil 420) is connected in parallel to the power supply 430. In particular, the capacitor bank 410, the first electromagnetic coil 420 and the power supply 430 are comprised in a first loop of the circuit 400. Furthermore, the switch 440 and the first LC circuit are connected in series to the power supply 430. In particular, the switch and the first LC circuit are comprised in a second loop of the circuit 400. More precisely, the switch 440, the capacitor bank 410 and the first electromagnetic coil 420 are comprised in the second loop of the circuit 400. In other embodiments, the arrangement of the capacitor bank 410, the first electromagnetic coil 420, the power supply 430, the switch 440 among the first loop and the second loop may be different.

The circuit 400 further comprises a resistance 450. Although the resistance 450 is shown in Figure 4 as a separate electronic component, in other embodiments the resistance may be integrated into the power supply 430. The resistance 450 and the switch 440 are connected in series to the power supply 430. The resistance 450 is comprised in a branch of the first loop of the circuit 400 such that the resistance 450 is connected to a positive pole of the power supply 430.

In other embodiments the resistance may be comprised in a branch of the first loop of the circuit 400 such that the resistance 450 is connected to a negative pole of the power supply 430. For example, the resistance 450 may be arranged such in the branch that the resistance 450 separates the power supply 430 (and/or the LC circuit) from the switch 440.

The resistance 450 of the circuit 400 may be smaller than 100 Ω, more preferably smaller than 50 Ω, even more preferably smaller than 20 Ω, most preferably smaller than 10 Ω.

In the embodiment of Figure 4, the capacitor bank 410 comprises a single capacitor.

Figure 4A illustrates a preferred embodiment of the capacitor bank 411. The capacitor bank 411 comprises a plurality of capacitors 412a, 412b, 412c, 412d, 412e, 414a, 414b, 414c, 414d, 414e, wherein the plurality of capacitors may be mutually connected by at least one parallel connection and at least one series connection. In particular, the capacitor bank 411 comprises two sets of capacitors 412 and 414 which are connected in series. The first set of capacitors 412 comprises five capacitors 412a, 412b, 412c, 412d, 412e which are connected in parallel. The second set of capacitors 414 comprises five capacitors 414a, 414b, 414c, 414d, 414e. The capacitor bank 411 may at least in part substitute the capacitor bank 410.

In other embodiments, the capacitor bank 411 may comprise more than the two capacitor sets 412 and 414 and/or the number of capacitors in each of the capacitor sets may be different. Alternatively, the capacitor bank 411 may only comprise one capacitor set 412. In general, the number of capacitors of a capacitor set 412, 414 may vary between 1 capacitor up to 20 capacitors, depending on the (magnetic stimulation) application.

The capacitor bank 411 allows for low internal resistance and flexibility. It is designed to minimize the internal equivalent series resistance (ESR) of the capacitor bank 411. In one of the embodiments, the ESR was reduced to 5 to 10 mΩ, depending on capacitor type(s) and model(s). By reducing internal resistance, the capacitor bank operates more efficiently, resulting in improved energy transfer and reduced power losses and heating.

The device offers flexibility in capacitor configurations, allowing for customization based on specific application requirements. For many applications, the capacitor bank 411 has been found to yield optimal performance.

In general, OTS capacitors rated 50 uF and 2,000V may offer an ESR higher than 10 mΩ, but are typically between 10 mΩ, and 20 mΩ. The capacitor bank 411 with 20 uF capacitors can be shown to be optimal because it gives lowest ESR, allows best air flow cooling of capacitors, extends lifetime of capacitors due total resonant current being split to several parallel connections and causes no internal damage to the individual capacitor electrodes where junctions of capacitor foil and connecting pins are made.

In general, to ensure proper cooling and temperature management of the capacitor banks 410, 411, the circuit 400, and/or the magnetic stimulation device comprising at least a part of the circuit 400, incorporates an innovative airflow cooling design. The capacitors of the capacitor bank 410, 411 are strategically positioned to facilitate efficient airflow around each individual component. This airflow cooling mechanism helps dissipate heat generated during operation, preventing overheating and ensuring the longevity of the capacitors. Additionally, the improved cooling design allows for the use of capacitors with higher power ratings, further enhancing the device's performance and reliability. Temperature sensors may be used to regulate the cooling power and, in case required, to limit or stop the device's operation.

The first switch 460 of the circuit 400 is configured for electrically opening and closing the first LC circuit. The first switch 460 and the first electromagnetic coil 420 are connected in series to the power supply 430. Furthermore, the first switch 460 and the capacitor bank 410 are connected in parallel to the power supply 430. For example, the first switch 460 is arranged in the second loop of the circuit 400.

The first switch 460 may be a semiconductor switch e.g., SCR, MOSFET, GAN-FET, SIC-FET, IGBT connected in a bipolar configuration, which is capable of switching high voltages in the range of 300 to 4,000 V. The electric current switching capability can be increased by parallel operation of such semiconductor switches such as first switch 460, enabling driving currents in the range of 100 to 1500 A. The voltage rating of a semiconductor switch such as first switch 460 can be increased by means of a series connection of individual semiconductor switches, as well. In general, both voltage rating and current rating of bipolar connected semiconductor switches such as first switch 460 depend on the (magnetic stimulation) application.

In general, opening and closing the first switch 460 may be based on at least two pulses. For example, the circuit 400 may be configured to trigger the first switch 460 based on a plurality of pulses. The plurality of pulses comprises a first pulse adapted to set a forward connection of the first switch 460 to a conductive state and a second pulse adapted to set a reverse connection of the first switch 460 to a conductive state. Therein, the second pulse may be generated after the first pulse, approximately at tᵣ/2, just before the current flowing through the first switch is about to reverse direction.

In general, the plurality of pulses may further comprise a third pulse adapted to set the reverse connection of the first switch 460 to the conductive state, wherein the third pulse is generated before the second pulse.

The use of such a plurality of pulses may be better understood with reference to Figures 5 and 6.

Figure 5A illustrates a circuit 500 for triggering the first switch 460 and/or the switch 440 based on optocouplers. Circuit 500 comprises phototriac optocoupler drivers and phototriac optocoupler 510a, 510b, semiconductor-controlled rectifier (SCR) 520a, 520b, input 530b (corresponding to element461 in Fig. 4) and output 530a (corresponding to element 462 in Fig. 4) of the first switch 460 configured to couple the circuit 500 to the circuit 400, trigger transformer driver 540a, 540b and a microcontroller 560.

Figure 5B illustrates a further circuit 600 for triggering the first switch 460 based on trigger transformers. The circuit 600 comprises trigger transformers 610a, 610b, semiconductor-controlled rectifiers (SCR) 620a, 620b, input 630b (corresponding to element 461 in Fig. 4) and output 630a (corresponding to element 462 in Fig. 4) of the first switch 460 configured to couple the circuit 600 to the circuit 400, trigger transformer drivers 640a, 640b and a microcontroller 660.

In the context of Figs. 5A and 5B, current flowing in a forward direction refers to current flowing from input 530b, 630b towards output 530a, 630a. Conversely, current flowing in a reverse direction refers to current flowing from output 530a, 630a towards input 530b, 630b.

In particular, triggering of the first switch 460 may comprise two pulses, wherein a first pulse ("Trig A") 550a, 650a determines the start of an oscillation of the first LC circuit and reverts forward SCR 520a, 620a into a conductive state. An example of a resulting current sine waveform is shown in Fig. 6. As can be seen from Fig. 6, the current sinewave starts with a certain delay relatively to a first pulse (cf. "Trig A" 550a, 650a as per Figs. 5A and 5B, respectively) since a certain time is required for the electric charge to enter the gate of forward SCR and fire it in conductive state.

By natural phenomena the SCR will remain open (self-sustained mechanism) until the voltage on the gate falls below a threshold voltage. The forward SCR may stop conducting immediately when the current through the first switch reverses direction and a voltage between an anode (connected to element 461 of Fig. 4) and a cathode (connected to element 462 of Fig. 4) of the forward SCR becomes negative. At exactly that time the reverse SCR may get positive voltage across an anode (connected to element 462 of Fig. 4) and a cathode (connected to element 461 of Fig. 4), the cathode fulfilling the first condition for firing. Before this happens, i.e., before the voltage across the forward SCR drops below its gate threshold voltage VT(TO), a second pulse (cf. "Trig B" 550b, 650b as per Figs. 5A and 5B, respectively) may be delivered to the gate of reverse SCR to transfer the electric charge, providing the final condition for firing.

To make operation of circuits 500, 600 more reliable the second pulse 550b, 650b may be triggered before half of a current oscillation of the first LC circuit is completed, e.g., before the current through the first switch 460 reverses its direction and voltage across forward SCR 520a, 620a drops below its gate threshold voltage VT(TO). Since the characteristics of the first LC circuit are known, the exact timing may be determined based on the corresponding resonant frequency fᵣ and/or the corresponding resonant period tᵣ. For example, for a given configuration of the first LC circuit, with tᵣ = 324 µs (as is the case in the embodiment of Fig. 6), pulses 650a, 650b with durations in the range of approximately 15 to 50 µs may be generated, and timing of the second pulse 650b may be approximately between 5 to 55 µs before the current through the first switch reverses its direction.

Furthermore, an additional third pulse (not illustrated in Figs 5A and 5B, but shown in Fig. 6) of a duration of approximately 15 to 50 µs may be additionally generated before the second pulse, at times approximately between 25 and 80 µs before the current through the first switch 460 reverses its direction, providing that a mutual distance (delay) between the third and second pulse is approximately in a range of 10 to 30 µs. This allows for a more secure triggering of the first switch 460 by minimizing the possibility of mis-triggering in case the earlier of the pulses did not deliver enough gate charge or if it was out of timing (e.g., was too early while a voltage between the anode and the cathode of SCR 520b, 620b was still negative). With the third pulse occurring too early there is no harm done to any component of the circuit 400 because the gate charge of reverse SCR 520b, 620b will shortly remain there, and it will contribute to transitioning the reverse SCR 520b, 620b to the conductive state. Generally speaking, SCRs are slow-switch devices, which this embodiment exploits. The more charge is injected into the gate the better the switch-on is achieved. For this reason, further additional pulses like the third pulse may be delivered to improve reliability.

Of course, the timing, i.e., duration and mutual distance in time (delay) between pulses such as pulses 550a, 650a and 550b, 650b, respectively, may depend on the configuration of a given LC circuit such as the first LC circuit, as the given configuration may determine the LC circuits resonant frequency fᵣ and/or the corresponding resonant period tᵣ.

In general, the duration of trigger pulses (tₚ) may be 0.05 tᵣ to 0.15 tᵣ. It should be taken onto account, however, that current SCR semiconductors require trigger pulse durations of at least 10-15 µs. The second pulse may be generated at times 0.015 tᵣ to 0.17 tᵣ before the half period, i.e., before the current through the first switch reverses its direction. And the third pulse may be generated at times 0.07 tᵣ - 0.25 tᵣ before the current through the first switch 460 reverses its direction, wherein the third and second pulse are separated by at most 0.15 tᵣ, preferably by at most 0.1 tᵣ, most preferably by at most 0.05 tᵣ.
as indicated above, the explanations concerning circuit 600 of Figure 5B may apply mutatis mutandis to circuit 500 of Figure 5A. In particular, trigger signals ("Trig A", "Trig B") 550a and 550b, as well as a third trigger signal (i.e., pulse) may be used to control circuit 500 of Figure 5A in the same manner as trigger signals ("Trig A", "Trig B") 650a and 650b, and a third trigger signal (i.e., pulse) may be used to control circuit 500 of Figure 5A. It is to be noted, however, that trigger pulse durations shorter than about 5 µs can be generated by optocouplers, but not by trigger transformers.

Returning to Figure 4, the switch 440 can be triggered in several ways. One way is analogue triggering which significantly simplifies circuit 400. For analogue triggering, the switch 440 acts as a reverse polarity voltage detector, e.g., by means of comparator circuit, and a corresponding high-voltage switch, which is triggered (opened) once a voltage of reverse polarity is detected.

Alternatively, the switch 440 may be triggered by a pulse, e.g., from a microcontroller via an optocoupler. In certain embodiments the pulse may be generated by microcontroller 560, 660 that is used for triggering the first switch 460 as described with reference to Figures 5 and 6. The pulse for controlling switch 440 may be of inverted logic, i.e.,, the pulse may be adapted such that the switch 440 electrically disconnects the first LC circuit from the power supply 430 when the first switch 460 electrically closes the first LC circuit. For example, the first pulse (trigger signal "Trig A" 550a, 650a) may be further adapted to instruct and/or cause the switch 440 to electrically disconnect the first LC circuit from the power supply 430.

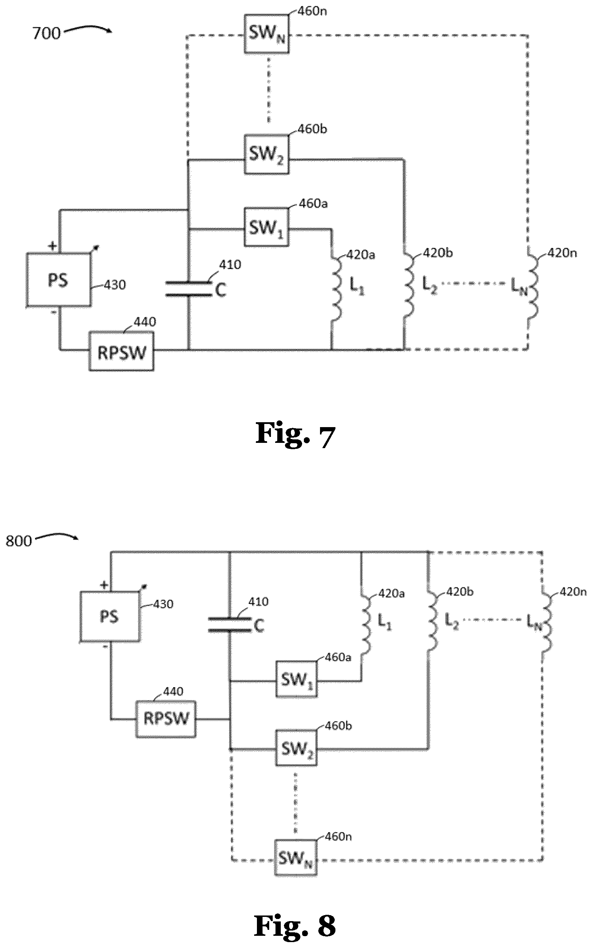

Figure 7 illustrates an exemplary embodiment of a multichannel circuit 700. The multichannel circuit 700 comprises the capacitor bank 410, the first electromagnetic coil 420a, further (second,..., n-th) electromagnetic coils 420b, ..., 420n, the power supply 430, the switch 440, the first switch 460a, and further (second,..., n-th) switches 460b, ..., 460n. The n-1 further electromagnetic coils 420b, ..., 420n and the capacitor bank 410 are arranged such that they form n-1 further (second, ..., n-th) LC circuits. Each of the n-1 further LC circuits comprises one of the n-1 further switches 460b, ..., 460n and each of the further switches 460b, ..., 460n is configured to electrically close and open the corresponding further LC circuit. Furthermore, the switch 440 is further configured to electrically disconnect any and/or each of the further LC circuits from the power supply 430 when a voltage across the capacitor bank 410 is reverse in polarity to the charging voltage, e.g., as defined with respect to the power supply 430.

Due to its small value and low power consumption the resistance 450 is not shown in Figure 7. It may be considered to be already integrated into (the printed circuit board of) the power supply 430. It is to be appreciated that according to the present invention the resistance 450, if there is any, may be connected to either the negative or positive pole of the power supply, and that a connection to the positive pole is a preferred choice when minimization of electromagnetic emissions and higher reliability are desired.

Figure 8 illustrates a further exemplary embodiment of a multichannel circuit 800. The multichannel circuit 800 comprises the capacitor bank 410, the first electromagnetic coil 420a, further (second,..., n-th) electromagnetic coils 420b, ..., 420n, the power supply 430, the switch 440, the first switch 460a and further (second, ..., n-th) switches 460b, ..., 460n. The n-1 further electromagnetic coils 420b, ..., 420n and the capacitor bank 410 are arranged such that they form n-1 further (second, ..., n-th) LC circuits. Each of the n-1 further LC circuits comprises one of the n-1 further switches 460b, ..., 460n and each of the further switches 460b, ..., 460n is configured to electrically close and open the corresponding further LC circuit. Furthermore, the switch 440 is further configured to electrically disconnect the any and/or each of the further LC circuits from the power supply 430 when a voltage across the capacitor bank 410 is reverse in polarity to the charging voltage, e.g., as defined with respect to the power supply 430.

Multichannel circuit 800 differs over multichannel circuit 700 in that the further switches 460b, ..., 460n separate the further electromagnetic coils 420b, ..., 420n from the switch 440. Meanwhile, in multichannel circuit 700, the further electromagnetic coils 420b, ..., 420n separate the further switches 460b, ..., 460n from the switch 440.

For example, the multichannel circuit 700, 800 may comprise the first electromagnetic coil 420a and three further (second, third, fourth) electromagnetic coils 420b, 420c, 420d, such that the multichannel circuit 700, 800 comprises four LC circuits, e.g., four channels. Furthermore, the multichannel circuit may comprise the first switch 460a and three further (second, third, fourth) switches 460b, 460c, 460d. Each of the switches 460a, 460b, 460c, 460d is configured for opening and closing the corresponding LC circuit and thereby activating and deactivating the corresponding channel. For example, the electromagnetic coils 420a, 420b, 420c, 420d may each be activated at a rate of 50 Hz. An activation rate of 50 Hz may be particularly optimal if the circuit 700, 800 is meant to be applied in muscle stimulation. For example, when the maximum available pulse repetition rate of the circuit 700, 800 is 200 Hz, this means that the four channels can be consecutively activated and deactivated at the switching rate of 200 Hz, resulting in substantially simultaneous stimulation with each of the four channels being activated at a repetition rate of 50 Hz.

It is to be appreciated that according to the present invention the switch 440 may be connected to either the negative or the positive pole of the power supply (a connection to the negative pole is shown in Figs. 7 and 8), and that a connection to the negative pole is a preferred choice when minimization of electromagnetic emissions and higher reliability are desired.

In general, as compared to the prior-art multichannel circuit 300 illustrated in Figure 3, the loss on the first and/or the further switches is two times smaller since there is only one switch that is activate during each activation of a channel.

Furthermore, in addition to higher electrical efficiency, an important advantage of the circuits 400, 700, 800, and/or devices comprising at least a part of such circuits, lies in higher achievable magnetic pulse repetition rates. This can be best appreciated by considering that magnetic stimulation devices commonly used for nerve stimulation typically operate at pulse repetition rates of up to 500 Hz and higher. However, using state-of-the-art technology, such devices, due to their low electrical efficiency, are limited to generating low-intensity magnetic fields at high pulse repetition rates. Using circuits such as circuits 400, 700, 800, it is possible to deliver more intense (higher- or high-intensity) magnetic stimulation while maintaining high pulse repetition rates, e.g., in the range of 100 to 500 Hz.

When considering the use of circuits such as circuits 400, 700, 800 for magnetic stimulation of muscles it is important to remember that clinically effective pulse repetition rates are limited by muscle fatigue. Evidence from repetitive peripheral magnetic stimulation at sufficient intensity to produce muscle contractions show that lower frequencies, e.g., f < 5 Hz, induce muscle twitching whereas higher frequencies, e.g., f >10 Hz, produce sustained muscle contractions due to temporal summation of a motor unit's recruitment. After prolonged stimulation, e.g., at least 10 minutes, at frequencies of 50 Hz or higher, muscle fatigue becomes a factor and the muscle response decreases. Consequently, muscle stimulation therapies are most commonly performed at pulse repetition rates of up to about 50 to 80 Hz.

Nevertheless, the higher repetition rates available with the circuits such as circuits 400, 700, 800 provide a significant advantage for performing muscle stimulation. This is because the therapy time can be substantially shortened, and the clinical outcome can be substantially improved when stimulation is performed over a larger body area. Since designing and powering a very large area single coil applicator is technically challenging, large-area stimulation may better be performed using a multichannel configuration, e.g., the circuit 700, 800, where individual electromagnetic coils 420a, 420b,..., 420n covering different body areas and/or corresponding muscles/muscle groups are repeatedly activated and deactivated at sufficiently high switching rates, with the maximum available switching rate being equal to the maximum available pulse repetition rate of the circuit. In this regard, the multichannel circuits 700, 800 represent a substantial advantage.

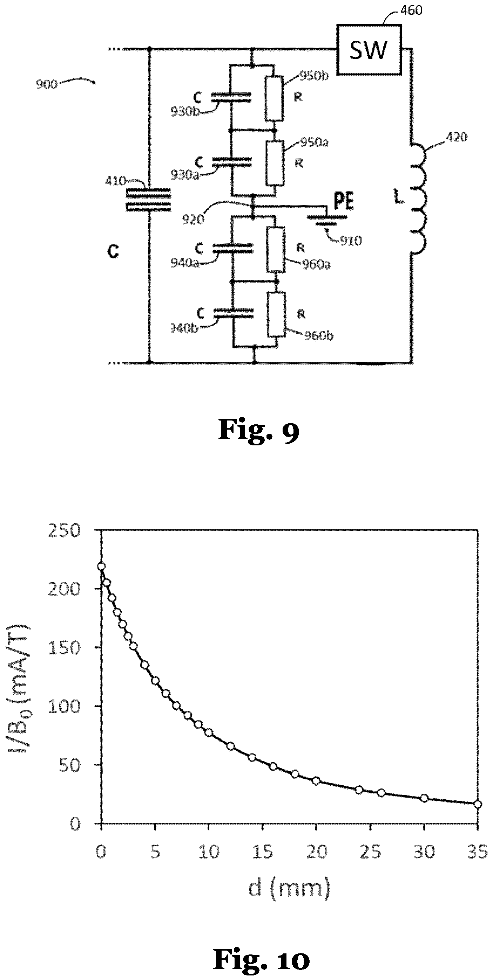

Figure 9 illustrates an exemplary embodiment of an RC network according to the present invention, as a part of a circuit 900 comprising RC pairs 930a and 950a, 930b and 950b, 940a and 960a, and 940b and 960b, and a connection to a protective earthing (PE) 910. The circuit 900 further comprises capacitor bank 410, the first electromagnetic coil 420 and the first switch 460 (all shown in Fig. 4). In addition, the circuit 900 further comprises a connection to protective earthing, wherein the connection to ground is electrically connected to ground 910 via a protective earthing (PE) node 920. In particular, the RC network and the capacitor bank 410 are connected in parallel to the power supply 430 and the first electromagnetic coil 420. The connection to ground PE protective earthing comprises multiple RC pairs of capacitors 930a, 930b, 940a, 940b and resistances 950a, 950b, 960a, 960b, wherein each RC pair capacitor 930a, 930b, 940a, 940b forms, together with the respective resistance 950a, 950b, 960a, 960b, a pair. For example, RC pair comprised of capacitor 930a and resistance 950a form a pair, etc.

These multiple RC pairs may collectively be referred to as an RC network herein. Preferably, as shown in Fig. 9, on each side of the PE node 920, the number of pairs coincides. For example, on an upper side of the PE node 920 there may be two pairs, namely capacitor 930a and resistance 950a as well as capacitor 930b and resistance 950b. Similarly, on a lower, i.e., opposite side of the PE node 920 there may be two pairs, namely capacitor 940a and resistance 960a as well as capacitor 940b and resistance 960b.

In other embodiments, directed to higher resonant circuit voltages, additional pairs of resistors and capacitors may be added to maintain more elements in series and in symmetry with respect to PE node 920.

The RC network may be used to reduce working voltages on safety isolating barriers inside the medical equipment that is, or may comprise, aspects of the present invention, in particular a circuit according to the present invention. Basic medical standards prohibit the presence of mains voltages, e.g., voltages derived from primary circuits in galvanic connection with the mains, on a patient. Thus, to provide for suitable isolation of the patient, safety barriers need to be provided towards mains parts, e.g., a minimum of two means of patient protection for working voltages occurring on a safety isolation barrier and towards secondary isolated circuits, and, e.g., typically one means of patient protection for working voltages occurring in isolation. Basic medical standards define quite strict requirements concerning clearance and creepage to cover also worst-case scenarios. Therefore, it is of great importance to achieve sufficiently low working voltages on all safety isolating barriers, which ensures standards compliance and results in better equipment performance.

This can be achieved by an RC network such as the RC network shown in Figure 9, which is symmetrically coupled to both poles of the capacitor bank 410. The RC network fixes floating potentials of the entire (first) LC circuit to ground 910. This solution nearly halves the working voltage between primary and patient and between patient and secondary. Therefore, smaller clearance and creepage distances may be (allowed to be) used.

Specifically, the resistance 450 may be connected in series to the positive pole of the power supply, since the positive pole is always impedance connected with lowest impedance towards a protective earthing, PE, through impedance network 930a, 930b and 950a, 950b of circuit 900. Generally, the RC network resistance lowers dU/dt transients and therefore reduces EMI emissions, thereby improves the performance and reliability of the circuit.

As already mentioned above, circuits such as circuits 400, 700, 800 may be particularly useful for muscle stimulation.

Muscle is a dynamic tissue implied in movement, posture, respiration, and corporal thermoregulation. It is considered to be the most abundant organ in the human body since it represents 30 to 50% of an individual's total corporal weight. Since the beginning of this millennium the concept of skeletal muscle has changed, moving from a concept of tissue whose only function was to generate strength and locomotion to one of an endocrine organ that produces metabolism-regulating proteins and that also performs other important functions.

Consequently, physical exercise has a number of beneficial biological effects. Muscle contraction, which activates skeletal muscular fibers, produces and releases several proteins called myokines, such as myostatin, musclin, irisin, interleukin-6 (IL-6), interleukin-1 (IL-1) and many others.

These myokines released into the bloodstream exert their effects in an either autocrine, paracrine, or endocrine manner, creating effects in different organs and tissues, including the brain, adipose tissue, bones, the liver, the gut, the pancreas, the vascular bed and the skin. For example, the exercise-induced acute increase in IL-6 stimulates an anti-inflammatory systemic environment. Thus, IL-6 promotes an increase in the production of other anti-inflammatory cytokines.

Lack of exercise provokes accumulation of more visceral fat and thereby further enhances inflammation and hence a network of chronic diseases. Thereby, a vicious cycle of chronic inflammation is established. Exercise training will lead to a decrease in visceral and cardiac fat mass and hence a decrease in circulating inflammatory molecules via a mechanism that involves exercise-induced increase in IL-6, as described above.

In general, most of the studies of the effects of muscle exercise on the release of myokines have focused on aerobic exercise, e.g., on cardiovascular exercise during which the patient's heart starts pumping, since this type of exercise is considered to be more effective for the production of myokines.

Nevertheless, anaerobic exercise through externally stimulated involuntary muscle contraction can exert positive effects on the release of various myokines, too, as has been demonstrated using electrically stimulated contractions of rat muscles.

However, anaerobic exercise is less effective in releasing myokines than aerobic exercise. This is because, for an efficient anaerobic treatment, a large enough muscle area needs to be stimulated. Circuits such as circuits 400, 700, 800 allow for stimulation of large muscle areas as they are extremely (electrically) efficient in generating time-varying magnetic fields.

Hence, especially - but expressly not exclusively - using circuits such as circuits 400, 700, 800, it becomes possible to reduce inflammation in a patient by means of magnetic stimulation. A suitable method may comprise applying a time-varying magnetic field to at least one muscle of the patient to magnetically stimulate the at least one muscle, wherein the magnetic field is adapted such as to trigger at least one contraction of the at least one muscle.

Such method may be used for managing chronic systemic inflammation (CSI), including reducing levels of insulin and TNF-α in blood. In general, the method may be used for reducing low- and/or medium-grade chronic inflammation. Application of a time-varying magnetic field may trigger and/or enhance myokine production in the patient, preferably by triggering at least one deep penetrating muscle contraction.

The time-varying magnetic field used in a method for reducing inflammation may be based at least in part on circuits such as circuits 400, 700, 800. In particular, using at least a part of a circuit such as circuit 400, 700, 800 provides patients with a powerful, effective, comfortable, non-invasive, motivating, painless, fast and safe method to obtain very strong muscle contractions without the need of patient exertion and with all the benefits of intense muscle activation.

However, it needs to be understood that such a method for reducing inflammation in a patient may equally well use any other circuit for generating the time-varying magnetic field. The present application is not limited to the use of a circuit such as circuit 400, 700, 800 in e disclosed methods for reducing inflammation.

In order to optimize the method and thus the reduction of (chronic) inflammation it must be evaluated what levels of (electro-)magnetic field are required for different muscle groups. This can be achieved by determining the dependence of a respectively induced current I (in mA) in given muscular tissue on the distance d between the electromagnetic coil and the targeted muscle tissue, and on the amplitude (Bₒ) of the magnetic field as generated in the near vicinity of the electromagnetic coil, e.g., at d ≈ 0 mm.

Figure 10 illustrates the dependence of the induced electrical current on the distance d, assuming a fixed amplitude (B₀) of the magnetic field and assuming a homogenous semi-infinite muscular tissue with electrical conductivity σ = 0.2 S/m.

In order to maximize the production of myokines, large muscle groups, such as those located in the arms, thighs, abdomen and buttocks may be targeted in the treatment sessions.

As illustrated in Table 1, the distance d of these muscles from the electromagnetic coil(s) and/or the applicator(s) positioned on the skin surface may depend on the muscle type, the sex and the obesity, which may be characterized by the body mass index (BMI) in kg/m².

**Table 1: Estimated distance of the body muscles from the applicator coil**

| | | Distance d (in mm) | | | |
|---|---|---|---|---|---|
| BMI (kg/m²) | Sex | Arm | Thigh | Abdomen | Buttock |
| < 25 | Female | 21.6 | 21.8 | 22.8 | 28.4 |
| | Male | 18.0 | 17.2 | 20.4 | 23.6 |
| 25-30 | Female | 24.2 | 24.2 | 27.6 | 30.7 |
| | Male | 19.8 | 18.6 | 24.4 | 25.3 |
| >30 | Female | 28.8 | 28.1 | 31.5 | 32.2 |
| | Male | 23.0 | 21.1 | 27.5 | 26.4 |

With respect to the data in Table 1, the distance d is defined as the distance to the half-thickness of the muscle, with the distance of the coil (d₀) from the skin surface being taken to be d₀ = 8 mm.

Considering the maximum amplitude for the magnetic field still guaranteeing patient safety, one may rely on studies which have shown that typical values of currents I induced during the stimulation of various muscles such as the gluteus maximus, quadriceps femoris, hamstrings, tibialis anterior and gastrocnemius are about 10 mA to 100 mA.

Table 2a illustrates values of the magnetic field B₀ required for I = 10 mA and Table 2b illustrates the values of the magnetic field B₀ required for I = 100 mA.

**Table 2a: Values of the magnetic field B₀ required for I = 10 mA**

| | | B₀ for I = 10 mA (in Gauss) | | | |
|---|---|---|---|---|---|
| BMI (kg/m²) | Sex | Arm | Thigh | Abdomen | Buttock |
| < 25 | Female | 247 | 250 | 268 | 397 |
| | Male | 191 | 181 | 227 | 283 |
| 25-30 | Female | 296 | 295 | 376 | 467 |
| | Male | 217 | 200 | 299 | 319 |
| >30 | Female | 409 | 390 | 492 | 518 |
| | Male | 271 | 237 | 373 | 344 |

**Table 2b: Values of the magnetic field B₀ required for I = 100 mA.**

| | | B₀ for I = 100 mA (in Gauss) | | | |
|---|---|---|---|---|---|
| BMI (kg/m²) | Sex | Arm | Thigh | Abdomen | Buttock |
| < 25 | Female | 2,466 | 2,500 | 2,675 | 3,972 |
| | Male | 1,913 | 1,809 | 2,269 | 2,829 |
| 25-30 | Female | 2,959 | 2,949 | 3,756 | 4,667 |
| | Male | 2,169 | 2,001 | 2,992 | 3,186 |
| >30 | Female | 4,090 | 3,896 | 4,920 | 5,183 |
| | Male | 2,710 | 2,371 | 3,729 | 3,442 |

Table 2a and Table 2b indicate that the preferred range of the magnetic field amplitudes for reducing inflammation in a patient depends on the muscle type, sex and BMI.

Generally, the production of anti-inflammatory myokines depends on the amount of contracted muscle mass and is hence strongly related to the amount of muscle mass exercised. Therefore, it is important to treat different large muscle groups jointly, covering areas from about 200 cm² to 3,000 cm².

This can be done particularly well using the above-described multichannel circuit, e.g., circuit 700 or circuit 800. For example, the multichannel circuit may comprise up to n=20 electromagnetic coils. The electromagnetic coils may be arranged such that they may be used for treating different large muscle groups jointly, covering areas from about 200 cm² to 3000 cm².

In general, when considering the stimulation of a muscle with a single electromagnetic coil, e.g., the electromagnetic coil 420 of circuit 400, and taking into account muscle fatigue, a preferred range for the pulse repetition rate r for generating myokine release is 3 Hz ≤ r ≤ 100 Hz, more preferably 5 Hz ≤ r ≤ 50 Hz, most preferably 10 Hz ≤ r ≤ 40 Hz.

However, preferably, a plurality of different muscles and/or muscle groups may be treated, (substantially) simultaneously, such as to increase the total amount of muscle exercised. Such stimulation of different muscles and/or muscle groups may be performed using the multichannel circuit comprising n electromagnetic coils 420a, 420b, ..., 420n as described above, e.g., circuit 700 or circuit 800. That said, the use if such a circuit is not compulsory. The treatment of chronic inflammation, and in particular the method and apparatus for reducing inflammation, as described herein may well work with other circuits. The present application is not limited in this regard.

In case of such (substantially) simultaneous stimulation, it should be ensured that the effective repetition rate (r_{eff}) as experienced by each electromagnetic coil 420a, 420b, ..., 420n and/or the respective targeted muscle and/or muscle group - and not necessarily the pulse repetition rate r characterizing the operation of the circuit - is in the preferred range 3 Hz ≤ r_{eff} ≤ 100 Hz, more preferably 5 Hz ≤ r_{eff} ≤ 50 Hz, most preferably 10 Hz ≤ r_{eff} ≤ 40 Hz. Meanwhile, the switching rate s, i.e., the rate of switching from one electromagnetic coil and/or corresponding applicator to another, is preferably in the range of 6 Hz ≤ s ≤ 2000 Hz, more preferably 10 Hz ≤ s ≤ 1000 Hz, most preferably 20 Hz ≤ s ≤ 800 Hz.

In some embodiments, one or more subsets of electromagnetic coils/applicators may be activated more frequently than other one or more subsets of electromagnetic coils/applicators, resulting in different effective repetition rates. Such differences may be meaningful depending on the respective targeted muscle type and the corresponding electromagnetic coil characteristics, e.g., size, shape, etc. The individual effective repetition rates of different subsets of electromagnetic coils/applicators may also vary during therapy, within a single treatment session or from one treatment session to the next, for example starting relatively low and gradually increasing.

Furthermore, (substantially) simultaneous stimulation may be applied to one or more subsets of electromagnetic coils/applicators only, e.g., only for electromagnetic coils/applicators placed over the abdominal area. Then, the effective switching rate experienced by different subsets of electromagnetic coils/applicators, may be smaller than the maximally achievable pulse repetition rate of the device and/or its circuit for generating a time-varying magnetic field.

Generally, the (peak/maximum) intensity of the magnetic field may vary as it is switched between different (subsets of) electromagnetic coils/applicators, with individual magnetic field values varying (being selected) depending on the patient, type of target muscle, target depth, and the corresponding electromagnetic coil characteristics, e.g., size, shape, etc. The intensity of the magnetic field may also vary during a therapy, within a single treatment session or from one treatment session to the next, for example starting relatively low and gradually increasing.

A clinical case series was conducted on 16 patients with a proven inflammatory profile confirmed by laboratory testing, e.g., pro/inflammatory cytokine TNF- α, and corresponding clinical symptoms were selected. Also, the chosen inflammation marker was correlated with fasting insulin levels for all patients prior to treatment. In addition, a patient questionnaire was completed to determine how much their ailments affect their lifestyle, wherein the questionnaire comprised a scale from 0 to 10, wherein 0 corresponds to being not affected and 10 to being extremely affected.

Patients' main reasons of consultation were persistent fatigue, insomnia, and low libido. Some patients had only one while others had a combination of these ailments. Average patient age was 56.7 years with an average BMI of 26.1.

The treatment consisted of 2 weekly treatment sessions for a total of 6 weeks, i.e., a total of 12 treatment sessions, wherein the time-varying magnetic field was generated using the above-described multichannel circuit, e.g., circuit 700 or circuit 800.

To maximize treatment output, the biggest and most powerful muscles in the body were selected to be targeted with intense stimulation. In particular, these muscles included the gluteus muscles, e.g., gluteus maximus, gluteus medius and gluteus minimus and the quadriceps.

For the treatment two radial applicators, each comprising an electromagnetic coil with a coil diameter of 110 mm and 19 coil windings, were applied on top of clothing.

Arranging the applicators on top of the clothing resulted in an average distance of the electromagnetic coil from the skin surface of about dₒ = 8 mm. The treatment was applied on the left and right glutes for 45 minutes each. Software presets to emulate intense resistance training were selected. Total power emission was gradually increased for patient comfort during the first few minutes, until 100% of the system's maximum power was reached.

This was followed by applying the same applicators with the same electromagnetic coils to the quadriceps for the same period of time. All patients comfortably tolerated the treatment which in no case needed to be interrupted. No undesirable side effects were recorded during or after the therapy.

Sixty days following the treatment session, patients were tested for their TNF-α and fasting insulin levels. A follow-up questionnaire was also completed to determine their current perception on how their ailments affected their lifestyle after completing the treatment. The results are summarized in Table 3.

| | | | | | | | | | | |
|---|---|---|---|---|---|---|---|---|---|---|
| | # of patients | Lifestyle Effect | | | TNF-α Level (pg/ml) | | | Fasting Insulin level (µU/ml) | | |
| | | Before | Post 60 | % var | Before | Post 60 | % var | Before | Post 60 | % var |

**Table 3: Results of the treatment sixty days following the treatment session.**

| | | | | | | | | | | |
|---|---|---|---|---|---|---|---|---|---|---|
| FATIGUE | 16 | 9.3 | 4.7 | -51% | 60.7 | 16.9 | -32% | 12.6 | 9.0 | -22% |
| INSOMNIA | 2 | 9.5 | 5.0 | -47% | 23.2 | 7.5 | -68% | 10.6 | 8.7 | 0% |
| LOW LIBIDO | 8 | 9.3 | 5.1 | -46% | 13.2 | 9.2 | -26% | 11.0 | 7.8 | -19% |

These clinical results show that the method for inflammation reduction described herein is highly effective.

It is to be appreciated that the inventive solutions presented herein may be useful for a broad range of magnetic stimulation applications. These applications include magnetic nerve stimulation therapies, muscle contraction stimulation therapies and therapies combing nerve stimulation and muscle contraction stimulation therapies. Magnetic nerve stimulation therapies, in particular spinal cord stimulation therapies, include relief of pain associated with failed back surgery syndrome, refractory angina pectoris, peripheral vascular disease, and complex regional pain syndrome. As to transcranial magnetic stimulation therapies, indications include depression, obsessive-compulsive disorder, migraines, anxiety and other brain-related conditions. Concerning muscle contraction stimulation therapies, the indications include muscle toning, firming and strengthening, improving muscle performance, fat reduction, urinary incontinence, stimulating neuromuscular tissue for bulk muscle excitation in the legs or arms for rehabilitative purposes, e.g., relaxation of muscle spasm, prevention or retardation of disuse atrophy, increasing local blood circulation, muscle re-education, immediate post-surgical stimulation of calf muscles to prevent venous thrombosis as well as maintaining or increasing a range of motion. It should be clear from the foregoing that, even though the expressions "therapy" and "indication" have been used (and will occasionally be used throughout the following), magnetic stimulation does not only serve therapeutic (in the more narrow sense), but also, additionally or alternatively, cosmetic ends.

In particular, and as already mentioned above, magnetic stimulation, specifically magnetic stimulation based on circuits such as circuits 400, 700, 800 may be particularly useful for treating erectile dysfunction and/or premature ejaculation and/or male infertility.

Erectile dysfunction may be defined by the difficulty of getting and maintaining an erection which is sufficient for satisfactory sexual intercourse. The erectile dysfunction reduces the well-being and life quality of the patient and his sexual partner. According to epidemiological studies, 96 % of the interviewed men in the age group 30-39 years, and 71,3 % of the interviewed men in the age group 70-80 years are sexually active. The prevalence of erectile dysfunction is age dependent 2 % (30-39 years), 10 % (40-49 years), 16 % (50-59 years), 34 % (60-69 years), 53 % (70-79 years). In the group of 40 to 70 years old randomly selected men, there is 52 % prevalence of erectile dysfunction for the entire group, subdivided in mild (17,2 %), moderate (25,2%) and severe (9,6%) erectile dysfunction. The prevalence of erectile dysfunction in France is 31,6 % in the group of men over 40 years of age. About 50 % of the patients with erectile dysfunction seek a therapy. Therefore, there is a need for methods and apparatuses for treating erectile dysfunction and/or premature ejaculation and/or male infertility.

The physiology of an erection may be broken down into arterial dilatation, relaxation of the cavernous bodies, and venous restriction. The sexual response cycle has four phases, given by excitement, plateau, orgasm, and resolution. The sexual arousal can start reflexogenic, i.e., direct stimulation of the tactile receptors in the genital area or psychogenic, i.e., through optic, acoustic, sensitive stimuli or fantasies. In both cases the erection reflex center S2-S4 in the spinal cord gets stimulated and sends impulses via the splanchnic nerves to the cavernous bodies of the penis.

The stimulation of the splanchnic nerves may increase the concentration of the nitric oxide (NO), thus leading to arterial dilatation, increasing the blood flow in the cavernous bodies and causing an erection. Through the arterial dilation the pressure inside the cavernous bodies will rise to average around 100 mm Hg in most men. Another factor helping to maintain tumescensce, i.e., erection, is the occlusion of the venous flow through the enlargement of the cavernous bodies.

The maximum rigidity is achieved through the contraction of the pelvic floor muscles, e.g., ischiocavernosus and bulbospongiosus. During this rigid erection phase through the contraction of the pelvic floor muscles the pressure within the erectile chambers can reach > 400 mmHg up to 1200 mmHg, which is 10 times higher than the systolic blood pressure.

If the arousal continues and becomes intensified the ejaculation and orgasmic center L2-L3 in the spinal cord may become stimulated. There are two phases in the process of ejaculation - emission - the process of deposing prostatic and vesicular gland secretion in the rear part of the urethra and the ejaculation itself - closing the internal bladder sphincter and through the contractions of bulbocavernosus muscle (one of the pelvic floor muscles) propelling the semen through the urethra, this process is controlled through somato-motoric nerve endings of the pudendal nerve.

Pelvic floor muscles are therefore very important in the process of erection reaching of 1200 mmHg pressure in the erectile chambers. Similarly, the process of ejaculation may depend on the contractions and strength of the bulbocavernosus muscle.

The sexual response may therefore be governed by the parasympathetic erection center S2-S4, e.g., the sacral or tail bone region, the sympathetic erection center T12-L2, e.g., the thoracic (upper) and lumbar (low-back) region, the ejaculation center L2-L3, e.g., the lumbar (low back) region, and the pelvic floor muscles. The S2-S4, i.e., the parasympathetic part is essential for the erection and is located about 15-20 cm measuring from the lowest part of the spinal column. The L2-L3, i.e., the center for ejaculation and orgasm, is located at 28-30 cm and the T12-L2, i.e., the sympathetic part of the erection center, is located about 36 - 40 cm measuring from the lowest part of the spinal column.

Therefore, the proposed method of magnetic stimulation for treating erectile dysfunction and/or premature ejaculation in a patient comprises stimulating nerves and/or nerve centers associated with at least one of the vertebrae Th12, L1, L2, L3, S2, S3 and/or S4 of a spinal column of the patient. Generally, the method of magnetic stimulation may further comprise treating male infertility.

The method, e.g., the corresponding magnetic field therapy, improves the erectile function of patients with erectile dysfunction and premature ejaculation. Clinical tests have shown that following 8 treatments with the proposed inventive method the erectile function improved by at least 25%, and the intravaginal ejaculation latency time (IELT) was observed to have doubled.

In particular, the novel magnet field therapy may act simultaneously on three main factors that determine the normal erectile function. First, on vessels, nerves and nerve centers in the spinal cord, e.g., S2-S4. Second, on T12-L2 sympathetic erectile reflex center and third on pelvic floor muscles. This therapy may increase the blood flow, may increase the elastin deposits in the cavernous bodies and may increase venous occlusion by strengthening the pelvic floor muscles.

Additionally, through the stimulation of the parasympathetic e.g., sacral region S2-S4, sympathetic reflex center, e.g., lumbar region L2-L3 and the pelvic floor muscles, a prolongation in the IELT may be achieved.

In a preferred embodiment, the method may be performed based on the above-described inventive circuit for generating a time-varying magnetic field. In some embodiments, the method may be performed based on other types of LC circuits and/or circuits adapted for generating a time-varying magnetic field. Alternatively, or in addition, the method may be performed based on a combination of the above-described inventive circuit and other types of LC circuits.

For example, the method may be performed based on two applicators. First, a sit applicator for simulating pelvic floor muscles, positioned bellow the pelvic floor. Second, a single back applicator for simultaneously stimulating the S2-S4 and/or L2-L4 and/or T12-L2 spinal cord areas as measured from the lowest part of the spinal column, positioned on the back substantially covering the above three spinal cord areas.

The single back applicator may be sufficiently large to substantially cover all three spinal cord areas extending from about 15 to about 40 cm of the spinal cord as measured from the lowest part of the spinal column. Preferably, the location of the back applicator may be centered at about 25-30 cm from the lowest part of the spinal column. The single back applicator may be circularly shaped with a preferred diameter (d) of the applicator d ≥ 25 cm. Alternatively, the single back applicator may be of an extended, e.g., elliptical, shape, which may comprise an effective diameter d in the extended direction of d ≥ 25 cm.

The sit applicator may as well be of a circular and/or extended shape. The preferred diameter of a circularly shaped sit applicator may be d = 25-35 cm in order to substantially stimulate most of the pelvic floor muscles.

In some embodiments, the single back applicator may be replaced by three back applicators, each covering one of the three spinal areas.

In another embodiment, consisting of two back applicators, the lower applicator may cover the spinal length of at least S2-S4 (d ≥ 6 cm), and the upper applicator may cover at least T12-L3 (d ≥ 8 cm).

Generally, the sit and one to three back applicators may be separate applicators to be positioned individually on the appropriate body parts. For example, the sit and back applicators may be fitted into a treatment chair.

When considering the required magnetic field values B₀ and pulse repetition rates (r) it is important to note that the function of the sit applicator is to stimulate muscles. The range of the required magnetic field amplitudes for the sit applicator may be substantially the same as those for the above-described method for treating inflammation, e.g., as shown in Tables 2a and 2b. Similarly, the preferred repetition rates for the sit applicator may be 3 Hz ≤ r ≤ 100 Hz, more preferably 5 Hz ≤ r ≤ 50 Hz, most preferably 10 Hz ≤ r ≤ 40 Hz.

The function of the back applicator is more complex since it represents a combination of nerve and muscle stimulation. For example, when maximal muscle stimulation in addition to nerve stimulation is desired, the preferred repetition rates for the back applicator may be 3 Hz ≤ r ≤ 100 Hz, more preferably 5 Hz ≤ r ≤ 50 Hz, most preferably 10 Hz ≤ r ≤ 40 Hz. However, when more intense nerve stimulation is desired, the preferred repetition rate may be 20 Hz ≤ r ≤ 400 Hz, more preferably 30 Hz ≤ r ≤ 300 Hz, most preferably 40 Hz ≤ r ≤ 200 Hz. In some embodiments, only one, i.e., primarily muscle or primarily nerve stimulation, or a combination of both, i.e., of primarily muscle and primarily nerve stimulation parameters, may be used.

The preferred range of the magnetic field for the back applicators may be concluded from Tables 2a and 2b for the case of male abdominal muscles for BMI ≤ 30 kg/m2, since the body mass index does not have a pronounced influence on the otherwise deeply located lower back muscles.

For a substantially simultaneous stimulation by one sit applicator and up to three back applicators, a multi-channel circuit configuration may be used, preferably being one of the above-described circuit for generating time-varying magnetic fields, e.g., the multi-channel configurations 700, 800 presented in Fig. 7 and Fig. 8. It is to be noted that by simultaneous stimulation it may be meant that an effective repetition rate (r_{eff}) as experienced by each individual coil applicator may be in the preferred range of the individual applicator, either for the primarily muscle or primarily nerve stimulation. On the other hand, for the simultaneous multi-channel stimulation the switching rate s, i.e., the rate of switching the activation from one applicator to another, may be in the preferred range of 6 Hz ≤ r ≤ 2000 Hz, more preferably 10 Hz ≤ r ≤ 1000 Hz, most preferably 20 Hz ≤ r ≤ 800 Hz.

It is important to appreciate that in some embodiments the value of the magnetic field Bₚ may change as the activation is being switched from one applicator to another depending on the muscle type and depth, and the corresponding applicator characteristics, e.g., size, shape, etc.

In some embodiments, certain subsets of applicators may be activated more frequently than the remaining subsets of applicators resulting in different effective repetition rates depending on the activated muscle type and the corresponding applicator characteristics, e.g., size, shape, etc. The individual magnetic field parameters, e.g., B and r, may also vary during a therapy, for example starting low and gradually increasing during a session.

Furthermore, simultaneous activation may be implemented only for the back applicators, and the effective switching rate experienced by the sit applicator may be smaller than the maximally achievable pulse repetition rate of the device.

In some embodiments, the effective repetition rate of at least one back applicator may be varied or alternating between two sets of parameters, in order to perform a combined primarily muscle and primarily nerve stimulation of the spinal nerves.

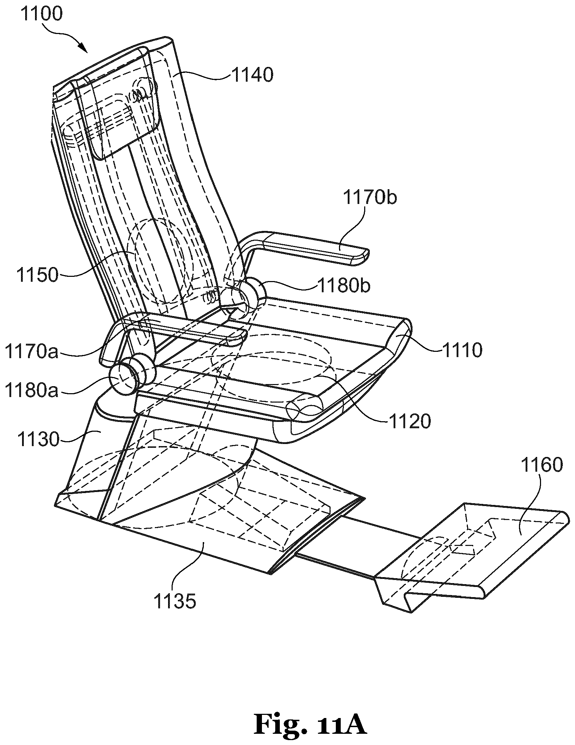

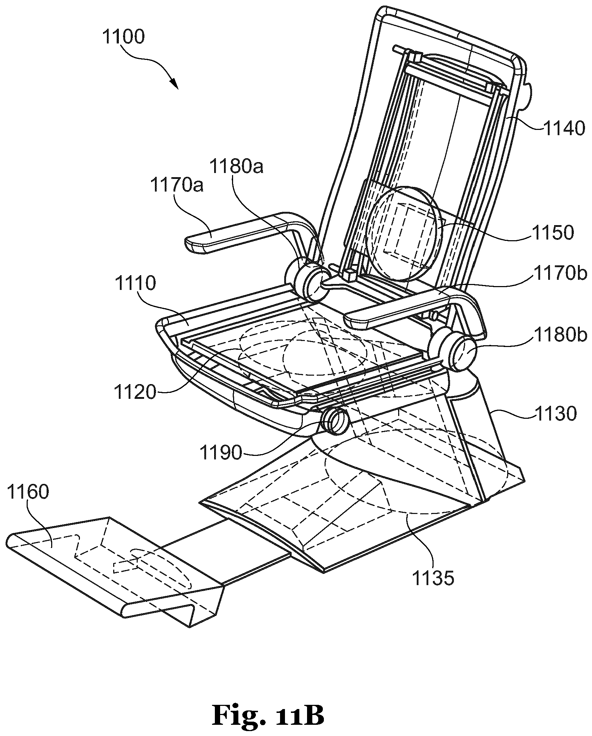

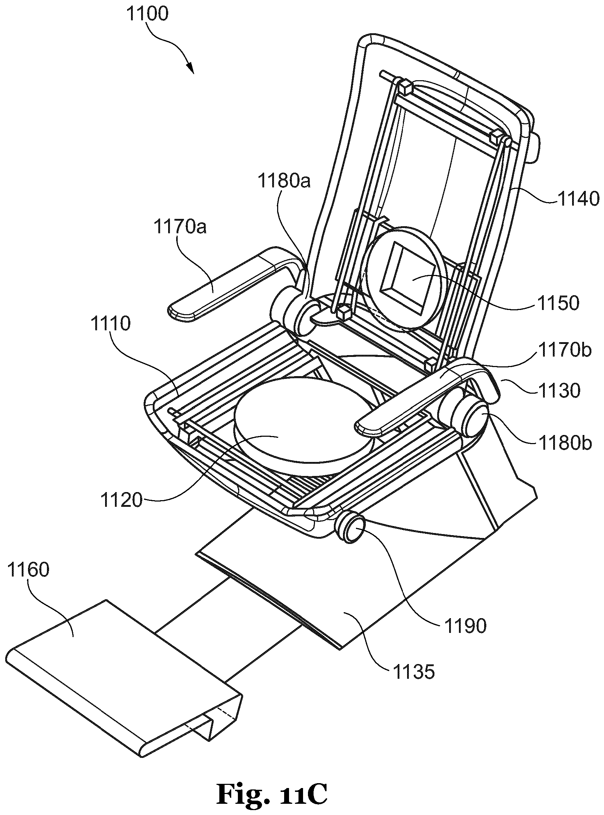

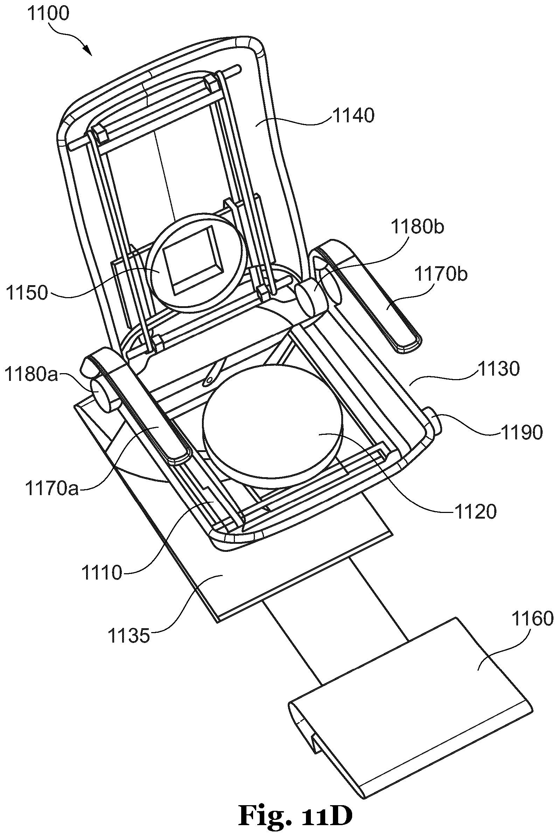

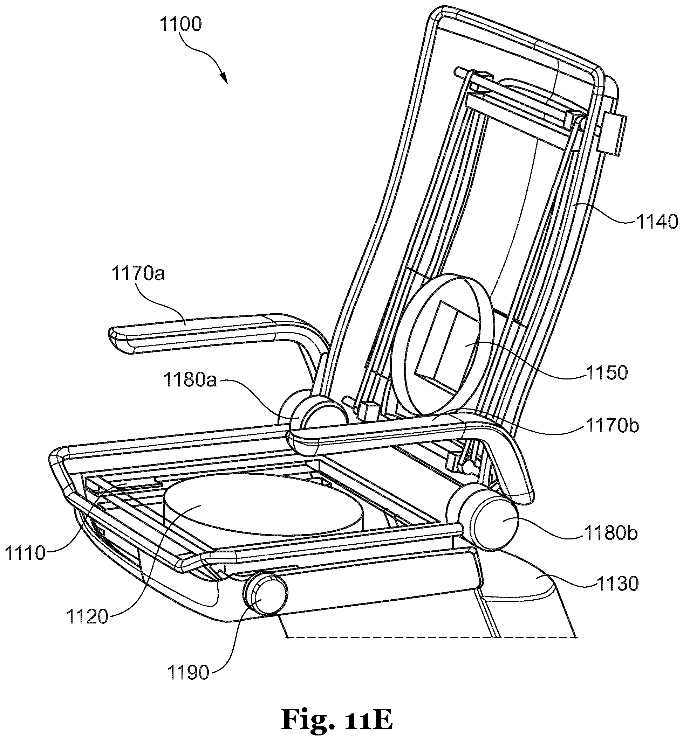

Figures 11A to 11E illustrate an exemplary embodiment of a chair 1100 for magnetic stimulation. The chair 1100 comprises a seating surface 1110 and an electromagnetic coil 1120, which is arranged at the seating surface 1110. The chair 1100 further comprises a frame 1130, wherein the frame 1130 is connected to a foot 1135. In addition, the chair 1100 comprises a backrest 1140, a second electromagnetic coil 1150 arranged at the backrest 1140, a footrest 1160 and armrests 1170a, 1170b. The armrests 1170a, 1170b are pivotably connected to the frame 1130 via the joints 1180a, 1180b.

The electromagnetic coil 1120 may comprise at least a part of the above-described circuit for generating a time-varying magnetic field for magnetic stimulation. For example, the electromagnetic coil may comprise the electromagnetic coil of the circuit 400 that at least partially forms the first LC circuit. In addition, or alternatively, the electromagnetic coil 1120 may comprise at least partially the above-described multichannel embodiment of the circuit for generating a time-varying magnetic field for magnetic stimulation. For example, the electromagnetic coil may comprise at least a part of the above-described circuit 700 or 800. Similarly, the second electromagnetic coil 1150 may comprise at least a part of the above-described circuit for generating a time-varying magnetic field for magnetic stimulation. For example, the second electromagnetic coil 1150 may comprise the electromagnetic coil of the circuit 400 that at least partially forms the first LC circuit. In addition, or alternatively, the second electromagnetic coil 1150 may comprise at least partially the above-described multichannel embodiment of the circuit for generating a time-varying magnetic field for magnetic stimulation. For example, the second electromagnetic coil 1150 may comprise at least a part of the above-described circuit 700 or 800.

Generally, the first and/or second electromagnetic coil 1120, 1150 may comprise a plurality of electromagnetic coils. For example, the first electromagnetic coil 1120 may comprise at least two electromagnetic coils and/or the second electromagnetic coil 1150 may comprise at least two electromagnetic coils. For example, the at least two electromagnetic coils of the first electromagnetic coil 1120 and/or the second electromagnetic coil 1150 may comprise at least partially the multichannel embodiment of the above-described circuit. For example, the at least two electromagnetic coils of the first electromagnetic coil 1120 may be part of a first multichannel circuit and the at least two electromagnetic coils of the second electromagnetic coil 1150 may be part of a second multichannel circuit. The first multichannel circuit may be independent of the second multichannel circuit. Alternatively, the first multichannel circuit and the second multichannel circuit are part of a multichannel circuit, e.g., the at least two electromagnetic coils of the first electromagnetic coil 1120 and the at least two electromagnetic coils of the second electromagnetic coil 1150 may be part of a joint multichannel circuit, e.g., a multichannel circuit comprising at least four electromagnetic coils.

Generally, if the chair 1110 at least partially comprises the above-described circuit for magnetic stimulation, the chair may only comprise a proper subset of the above-described circuit. For example, the chair 1110 may comprise at least a part of the electromagnetic coils of the circuit, e.g., the electromagnetic coils 420a, ..., 420n, but may not comprise a capacitor bank, e.g., the capacitor bank 410, and/or a power supply for the circuit, e.g., the power supply 430, and/or the switches 460a, ..., 460n, and/or the resistance, e.g., the resistance 450, and/or the first switch 440. In some embodiments, the chair 1110 may only comprise at least a part of the electromagnetic coils, e.g., the electromagnetic coils 420a, ..., 420n. For example, the parts of the circuit not comprised in the chair 1110 may be part of a control and/or supply device of the chair 1110. The control and/or supply device of the chair 1110 may be an external device, e.g., a device separated from the chair 1110. A device separated from the chair may comprise a device that is movable independent of the chair 1110 and/or which only connects to the chair by at least one wire. In particular, the at least one wire may be configured such as to deliver an electrical current from the external device to the electromagnetic coils of the chair 1110.

The foot 1135 may be configured to hold and/or to stabilize the frame 1130. In some embodiments, the foot may be adapted to be fixed to a ground. The size of the foot 1135 may generally depend at least partially on the size of the seating surface. For example, the foot 1135 may be such that the size of the foot at least partially comprises a projection of the seating surface 1110 onto the foot 1135. In particular, the size of the foot 1135 may be such as to comprise the projection of the seating surface 1110 onto the foot 1135. The foot 1135 may comprise a material comprising a metal. For example, the material of the foot 1135 may comprise aluminum. In addition, or alternatively, the foot 1135 may comprise plastic.

Arranging an electromagnetic coil 1120 at the seating surface 1110 may comprise arranging the electromagnetic coil below the seating surface 1110 and/or within a cushioning of the seating surface 1110. Alternatively, or in addition, arranging the electromagnetic coil 1120 at the seating surface may comprise arranging the electromagnetic coil 1120 below the cushioning of the seating surface 1110. Generally, the electromagnetic coil 1120 may be arranged essentially parallel to the seating surface 1110 of the chair 1100. In some embodiments, the electromagnetic coil may be arranged with an inclination with respect to the seating surface. For example, the inclination with respect to the seating surface may comprise an angle of at most 45°, at most 30°, at most 25°, at most 20°, or at most 15°. In some embodiments, the inclination of the electromagnetic coil 1120 with respect to the seating surface may be adjustable. For example, the chair may be adapted such that the inclination of electromagnetic coil 1120 may be changed from a first inclination to a second inclination. Arranging an electromagnetic coil 1120 at the seating surface 1110 may comprise arranging the electromagnetic coil 1120 at a predefined distance from the seating surface. For example, the distance of the electromagnetic coil 1120 from the seating surface 1110 and/or the cushioning of the seating surface 1110 may be at most 80 cm, preferably at most 60 cm, more preferably at most 40 cm, most preferably at most 20 cm. In addition, or alternatively, the distance between the electromagnetic coil 1120 and the seating surface 1110 and/or the cushioning of the seating surface may adjustable. For example, the chair 1100 may be adapted such that the electromagnetic coil 1120 may be moved from a first distance with respect to the seating surface 1110 and/or the cushioning of the seating surface 1110 to a second distance with respect to the seating surface 1110 and/or the cushioning of the seating surface 1110.

The footrest 1160 extends from the frame 1130 and/or the foot 1135 via a connector. The footrest 1160 is adapted to receive at least one foot of the patient. In general, the footrest 1160, particularly the surface of the footrest 1160, may be inclined with respect to the foot 1135 and/or the seating surface 1110. For example, the footrest 1160 may be arranged such at the connector that the inclination of the surface of the footrest may be adjustable. In addition, the connector may be such that a distance between the footrest 1160 and the seating surface 1110 and/or the frame 1130 and/or the foot 1135 is adjustable. By adjusting the inclination and/or the distance of the footrest 1160 the footrest 1160 can be adapted to the individual needs of the patient, thus enhancing the comfort of the patient during the treatment. In some embodiments, the footrest 1160 comprises at least one electromagnetic coil.

Furthermore, the backrest 1140 may be adjustable with respect to the seating surface 1110 and/or with respect to the frame 1130 and/or with respect to the foot 1135. For example, the backrest 1140 may be pivotable with respect to the seating surface 1110. In particular, the backrest 1140 may be pivotably connected to the seating surface 1110 via the joints 1180a, 1180b. In some embodiments, separate joints may be used to pivotably connect the backrest 1140 with the seating surface 1110 and/or the frame 1130. By pivoting the backrest 1140 with respect to the seating surface 1110 an inclination between the backrest 1140 and the seating surface 1110 may be adjusted. Therefore, the backrest 1140 can be adapted to the individual needs of the patient, thus enhancing the comfort of the patient during the treatment.

The position of the seating surface 1110 relative to the first electromagnetic coil 1120 can be adjusted. For example, the position of the seating surface 1110 may be adjusted relative to the first electromagnetic coil 1120 with respect to one direction. In some embodiments, the seating surface may be adjusted relative to the first electromagnetic coil 1120 with respect to two and/or three directions. Generally, the position of the seating surface may be controlled and/or adjusted by the rotatable hand controller 1190. For example, the position of the seating surface with respect to one direction may be adjusted by (mechanically) rotating the hand controller 1190. In particular, the position of the seating surface may be adjusted in a direction and/or axis essentially parallel to the seating surface. In some embodiments, the hand controller 1190 may be an electronical hand controller. For example, the hand controller 1190 may comprise an electronic switch. In particular, by actuating the switch the position of the seating surface 1120 may be adjusted. In some embodiments, the frame 1130 may comprise rails on which the seating surface 1120 is put on. For example, the rails may be such that the seating surface 1120 is slidable with respect to the rails. In particular, a slidable seating surface 1120 may enable the adjustment of the seating surface 1120 with respect to at least one direction.

In some embodiments, adjusting the position of the seating surface 1110 relative to the first electromagnetic coil 1120 may comprise not adjusting the position of the backrest 1150 with respect to the first electromagnetic coil 1120. For example, the position of the seating surface 1110 relative to the first electromagnetic coil 1120 may be adjusted independently of the position of the backrest 1140 relative to the first electromagnetic coil 1120.

Alternatively, the adjusting the position of the seating surface 1110 relative to the first electromagnetic coil 1120 may comprise adjusting the position of the backrest 1150 with respect to the first electromagnetic coil 1120. For example, moving the position of the seating surface 1110 relative to the first electromagnetic coil 1120 may induce moving the position of the backrest 1140 relative to the first electromagnetic coil 1120. For example, inducing the moving of the position of the backrest 1140 relative to the first electromagnetic coil 1120 may comprise a connection between at least a part of the seating surface 1110 and at least a part of the backrest 1140.

In general, the first electromagnetic coil 1120 may be non-movably attached to the frame 1130. For example, the first electromagnetic coil 1120 may be attached to the frame by means of screws. Further, the first electromagnetic coil 1120 may be placed symmetrical with respect to the seating surface 1110. Placing the first electromagnetic coil 1120 symmetrical with respect to the seating surface 1110 may comprise that at least with respect to one direction the first electromagnetic coil 1120 and the seating surface 1110 are symmetric. For example, when the seating surface 1110 moves relative to the first electromagnetic coil 1120, the first electromagnetic coil 1120 may conserve the symmetric arrangement with respect to the direction of the movement.

In general, the first electromagnetic coil 1120 may adapted to stimulate nerves and/or nerve centers. For example, the first electromagnetic coil may be adapted to stimulate nerves and/or nerve centers associated with at least one of the vertebrae S₂, S₃ and/or S₄ of the spinal column of the patient. In addition, or alternatively, the first electromagnetic coil 1120 may be adapted to stimulate at least a part of the pelvic floor of the patient. In addition, or alternatively, the first electromagnetic coil 1120 may be arranged and/or placed under the penis of the patient.

The second electromagnetic coil 1150 is arranged at the backrest 1140. The second electromagnetic coil 1150 may be adjustable such as to move relative to the backrest 1140. For example, the second electromagnetic coil may be movable relative to the backrest 1140 with respect to one direction. For example, the second electromagnetic coil 1150 may be movable with respect to the main elongation direction of the backrest 1140. In some embodiments, the second electromagnetic coil 1150 may be movable relative to the backrest 1140 with respect to two and/or three directions. Generally, the second electromagnetic coil may be arranged such that pivoting the backrest 1140 also pivots the second electromagnetic coil 1150.

Arranging the second electromagnetic coil 1150 at the backrest 1140 may comprise arranging the electromagnetic coil 1150 behind the backrest 1140 and/or within a cushioning of the backrest 1140. Alternatively, or in addition, arranging the second electromagnetic coil 1150 at the backrest 1140 may comprise arranging the second electromagnetic coil 1150 behind the cushioning of the backrest 1140. Generally, the electromagnetic coil 1150 may be arranged essentially parallel to the backrest 1140 of the chair 1100. In some embodiments, the second electromagnetic coil 1150 may be arranged with an inclination with respect to the backrest 1140. For example, the inclination with respect to the backrest 1140 may comprise an angle of at most 45°, at most 30°, at most 25°, at most 20°, or at most 15°. In some embodiments, the inclination of the backrest 1140 with respect to the second electromagnetic coil 1150 may be adjustable. For example, the chair may be adapted such that the inclination of second electromagnetic coil 1150 may be changed from a first inclination to a second inclination. Arranging the second electromagnetic coil 1150 at the backrest 1140 may comprise arranging the second electromagnetic coil 1150 at a predefined distance from the backrest 1140. For example, the distance of the second electromagnetic coil 1150 from the backrest 1150 and/or the cushioning of the backrest 1150 may be at most 80 cm, preferably at most 60 cm, more preferably at most 40 cm, most preferably at most 20 cm. In addition, or alternatively, the distance between the second electromagnetic coil 1150 and the backrest 1140 and/or the cushioning of the backrest 1140 may adjustable. For example, the chair 1100 may be adapted such that the second electromagnetic coil 1150 may be movable from a first distance with respect to the backrest 1140 and/or the cushioning of the backrest 1140 to a second distance with respect to the backrest 1140 and/or the cushioning of the backrest 1140.

In general, the second electromagnetic coil 1150 may be adapted to stimulate a lower part of the back of the patient. For example, the second electromagnetic coil 1150 may be adapted to stimulate nerves and/or nerve centers associated with at least one of the vertebrae Th12, L1, L2, and/or L3. In addition, the second electromagnetic coil 1150 may be adapted to stimulate nerves and/or nerve centers associated with at least one of the vertebrae S2, S3 and/or S3 of the spinal column of the patient. The second electromagnetic coil 1150 may be adapted to stimulate at least a part of an area located at least 5 cm, preferably at least 10 cm, most preferably at least 15 cm from the lowest part of the spinal column. In addition, or alternatively, the second electromagnetic coil may be adapted to stimulate a part of an area located at most 60 cm, preferably at most 50 cm, most preferably at most 40 cm from the lowest part of the spinal column.

## Claims

1. An apparatus for magnetic stimulation, comprising:
a first element adapted to receive at least a part of a body of a patient; and
a first set of one or more magnetic coils arranged at the first element; wherein the first element is adjustable such as to move relative to the first set of one or more magnetic coils.

2. The apparatus according to claim 1, further comprising a frame, wherein:
the first element is movably attached to the frame; and
the first set of one or more magnetic coils is non-movably attached to the frame.

3. The apparatus according to claim 1 or 2, wherein the apparatus is adapted to receive and/or support the patient in a sitting or lying position.

4. The apparatus according to any of the preceding claims, wherein the apparatus forms at least a part of a chair, the chair further comprising:
a second element adapted to receive at least a part of the body of the patient;
wherein the first element forms at least a part of a seating surface of the chair and the second element forms at least a part of a backrest of the chair; or
wherein the first element forms at least a part of the backrest of the chair and the second element forms at least a part of the seating surface of the chair.

5. The apparatus according to claim 4, wherein the apparatus further comprises:
a second set of one or more magnetic coils arranged at the second element.

6. The apparatus according to claim 5, wherein:
the second element is adjustable such as to move relative to the second set of one or more magnetic coils; and/or
at least one magnetic coil of the second set is adjustable such as to move relative to the second element.

7. The apparatus according to any of the preceding claims, wherein the apparatus, in particular at least one magnetic coil, is adapted to stimulate a lower part of the back of the patient.

8. The apparatus according to any of the preceding claims, wherein the apparatus, in particular at least one magnetic coil, is adapted to stimulate nerves and/or nerve centers associated with at least one of the vertebrae Th12, L1, L2, L3, S2, S3 and/or S4.

9. The apparatus according to any of the preceding claims, wherein the first set and/or the second set comprises two or more magnetic coils.

10. The apparatus according to any of the preceding claims, wherein:
a first magnetic coil is adapted to stimulate nerves and/or nerve centers associated with at least one of the vertebrae Th12, L1, L2 and/or L3 of a spinal column of the patient; and
a second magnetic coil is adapted to stimulate nerves and/or nerve centers associated with at least one of the vertebrae S2, S3 and/or S4 of the spinal column of the patient.

11. The apparatus according to claim 10, wherein:
the first magnetic coil is adapted to stimulate at least a part of an area located at least 10 cm, preferably at least 20 cm, most preferably at least 28 cm and/or at most 60 cm, preferably at most 50 cm, most preferably at most 40 cm from the lowest part of the spinal column; and/or
the second magnetic coil is adapted to stimulate at least a part of an area located at least 5 cm, preferably at least 10 cm, most preferably at least 15 cm and/or at most 40 cm, more preferably at most 30 cm, most preferably at most 20 cm from the lowest part from the spinal column.

12. The apparatus according to claim 10 or 11, wherein:
the first coil is adapted to stimulate an area of at least 8 cm width; and/or
the second coil is adapted to stimulate an area of at least 6 cm width.

13. The apparatus according to any of the claims 1 to 9,
wherein a first magnetic coil is adapted to stimulate nerves and/or nerve centers associated with at least one of the vertebrae Th12, L1, L2 and/or L3 as well as nerves and/or nerve centers associated with at least one of the vertebrae S2, S3 and/or S4 of a spinal column of the patient;
wherein the first magnetic coil is preferably adapted to stimulate at least a part of an area located at least 5 cm, preferably at least 10 cm, most preferably at least 15 cm and/or at most 60 cm, preferably at most 50 cm, most preferably at most 40 cm from the lowest part of the spinal column; and/or
wherein the first magnetic coil is preferably adapted to stimulate an area of at least 10 cm, preferably at least 15 cm, most preferably at least 20 cm and/or at most 40 cm, preferably 30 cm, most preferably at most 22 cm width.

14. The apparatus according to claim 13, wherein the first magnetic coil comprises an elongated, preferably elliptical, shape.

15. Method of magnetic stimulation, preferably for treating erectile dysfunction and/or premature ejaculation in a patient, comprising:
stimulating nerves and/or nerve centers associated with at least one of the vertebrae Th12, L1, L2, L3, S2, S3 and/or S4 of a spinal column of the patient.
